# EUROPÄISCHE PATENTANMELDUNG

(11) **EP 4 681 686 A2**
(43) Veröffentlichungstag der Anmeldung: **21.01.2026**
(21) Anmeldenummer: 25220064.7
(22) Anmeldetag: 06.10.2017
(51) Int. Cl.: A61F 2/74

(54) **AKTUATOR-DÄMPFER-EINHEIT**

(30) Priorität: 06.10.2016 DE 102016118999
(62) Teilanmeldung aus: 23172121.8
(71) Anmelder: Otto Bock Healthcare Products GmbH, 1110 Wien (AT)
(72) Erfinder: Die Erfindernennung liegt noch nicht vor
(74) Vertreter: Gramm, Lins & Partner Patent- und Rechtsanwälte PartGmbB

(57) **Zusammenfassung**

Die Erfindung betrifft eine Aktuator-Dämpfer-Einheit zum Einsatz in orthetischen oder prothetischen Vorrichtungen (1, 2, 3; 300), mit einem an der Vorrichtung (1, 2, 3, 300) festlegbaren Gehäuse (10), in dem ein Zylinder (12) ausgebildet ist, in dem ein erster Kolben (30) verlagerbar gelagert und mit einer Kolbenstange (35) gekoppelt ist, die mit einem ersten Ende (351) an dem ersten Kolben (30) angeordnet und mit einem zweiten Ende (352) mit der orthetischen oder prothetischen Vorrichtung (1, 2, 3, 300) koppelbar ist, wobei der erste Kolben (30) zwei Fluidkammern (41, 42) in dem Zylinder (12) voneinander trennt und eine Kolben-Zylinder-Einheit (100) bildet, wobei zumindest ein weiterer Kolben (32, 33) in dem Zylinder (12) verlagerbar angeordnet und mit dem ersten Kolben (30) zur Ausbildung zumindest einer weiteren, volumenveränderbaren Fluidkammer (43, 44, 45, 46) über einen komprimierbaren Energiespeicher (50) zwischen den Kolben (30, 32, 33) gekoppelt ist.

## Beschreibung

Die Erfindung betrifft eine Aktuator-Dämpfer-Einheit zum Einsatz in orthetischen oder prothetischen Vorrichtungen, mit einem an der Vorrichtung festlegbaren Gehäuse, in dem ein Zylinder ausgebildet ist, in dem ein erster Kolben verlagerbar gelagert und mit einer Kolbenstange gekoppelt ist, die mit einem ersten Ende an dem ersten Kolben angeordnet und mit einen zweiten Ende mit der orthetischen oder prothetischen Vorrichtung koppelbar ist, wobei der erste Kolben zwei Fluidkammern in dem Zylinder voneinander trennt und eine Kolben-Zylinder-Einheit bildet. Unter orthetischen oder prothetischen Vorrichtungen werden insbesondere Orthesen, Prothesen und Exoskelette verstanden. Die Aktuator-Dämpfer-Einheit kann auch in der Robotik eingesetzt werden.

Bei orthetischen oder prothetischen Vorrichtungen werden häufig zwei Komponenten relativ zueinander verlagert, beispielsweise werden ein Oberteil und ein Unterteil um eine Gelenkachse bei einem Orthesengelenk oder Prothesengelenk relativ zueinander verschwenkt, ebenso können längsverschiebliche Relativbewegungen zwischen zwei orthetischen oder prothetischen Komponenten auftreten. Häufig ist es notwendig, die Relativbewegung zu dämpfen. Zu diesem Zweck werden hydraulische und/oder pneumatische Dämpfer eingesetzt, die eine Extension oder Flexion eines Drehgelenkes oder einer Verschiebebewegung in die eine oder andere Richtung dämpfen. Die Dämpfer können einstellbar ausgestaltet sein, um auf der Grundlage von Sensordaten oder festgelegten Steuerkurven über den Verlauf einer Bewegung veränderte Widerstände gegen die Relativbewegung bereitzustellen. Hierzu sind in Strömungskanälen Ventile oder Drosseln vorgesehen, die gegebenenfalls einstellbar sind.

Um eine Bewegung der Komponenten der orthetischen oder prothetischen Vorrichtung zu unterstützen, sind Aktuatoren vorgesehen, die beispielsweise als Elektromotoren ausgebildet sein können. Ebenfalls können hydraulische oder pneumatische Antriebe vorgesehen sein. Über Energiespeicher ist es möglich, kinetische Energie zu speichern, so dass im weiteren Verlauf des Bewegungszyklusses oder zu einem anderen Zeitpunkt diese gespeicherte Energie, die beispielsweise durch Abbremsen einer Flexionsbewegung erhalten wurde, wieder dem System zugeführt werden kann. Über die Rückführung der Energie wird die orthetische oder prothetische Vorrichtung angetrieben, so dass ein Aktuator vorliegt. Über einen Aktuator wird Energie einer Bewegung unterstützend zugeführt.

Aus der DE 10 2012 013 141 A1 ist eine orthetische oder prothetische Gelenkeinrichtung mit einem Oberteil und einem schwenkbar daran angeordneten Unterteil mit zumindest einer Hydraulikeinheit zwischen dem Oberteil und dem Unterteil bekannt, die einen in einem Gehäuse mit einer Extensionskammer und Flexionskammer beweglichen Kolben aufweist, der mit dem Oberteil oder dem Unterteil gekoppelt ist. Über eine Druckbereitstellungseinrichtung kann der Kolben mit einem Druck beaufschlagt werden, wobei die Druckbereitstellungseinrichtung zumindest einen Druckspeicher aufweist, der über eine Umschalteinrichtung wahlweise mit der Extensionskammer oder mit der Flexionskammer koppelbar ist. Der Druckspeicher kann mit einer Pumpe gekoppelt sein, um über die Pumpe den Druckspeicher aufzufüllen. Nachteilig an einer solchen Konstruktion ist der vergleichsweise hohe Raumbedarf durch den externen Druckspeicher.

Aufgabe der vorliegenden Erfindung ist es daher, eine Aktuator-Dämpfer-Einheit bereitzustellen, die variabel einsetzbar, robust und kompakt ist.

Erfindungsgemäß wird diese Aufgabe durch eine Aktuator-Dämpfer-Einheit mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die Aktuator-Dämpfer-Einheit zum Einsatz in orthetischen oder prothetischen Vorrichtungen, mit einem an der Vorrichtung festlegbaren Gehäuse, in dem ein Zylinder ausgebildet ist, in dem ein erster Kolben verlagerbar gelagert und mit einer Kolbenstange gekoppelt ist, die mit einem ersten Ende an dem ersten Kolben angeordnet und mit einem zweiten Ende mit der orthetischen oder prothetischen Vorrichtung koppelbar ist, wobei der erste Kolben zwei Fluidkammern in dem Zylinder voneinander trennt oder eine Kolben-Zylinder-Einheit bildet, sieht vor, dass zumindest ein weiterer Kolben mit dem ersten Kolben zur Ausbildung zumindest einer weiteren, volumenveränderbaren Fluidkammer gekoppelt ist. Die Kopplung kann mechanisch oder fluidtechnisch, insbesondere hydraulisch erfolgen. Der zweite Kolben ist bevorzugt in dem Zylinder verlagerbar angeordnet und bildet zumindest eine weitere, volumenveränderbare Fluidkammer aus. Durch den zweiten Kolben, der in einer Weiterbildung der Erfindung innerhalb des Zylinders verlagerbar angeordnet ist, ist es möglich, einer weitere, dritte Fluidkammer zur Speicherung mechanischer Energie zu nutzen, so dass neben einer durch beispielsweise Drosselventile gesteuerten Bewegungsdämpfung bei einer kontrollierten Abgabe der gespeicherten Energie zu einem beliebigen Zeitpunkt die reine Dämpferfunktion um eine Aktuatorfunktion erweitert werden kann. Durch die Ausbildung der dritten Fluidkammer mit dem veränderlichen Fluidvolumen können zusätzliche Möglichkeiten zur Veränderung der Dämpfungseigenschaften bereitgestellt werden. Diese erweiterten Möglichkeiten lassen sich mit der Option einer Bewegungsunterstützung kombinieren. Die Speicherung überschüssiger Energie erfolgt bei Anwendungen in der Prothetik oder Orthetik, insbesondere bei Prothesen oder Orthesen der unteren Extremität, in denjenigen Phasen eines Bewegungszyklusses, in denen ein Energieüberschuss herrscht, also wenn eine Bewegung abgebremst oder angehalten werden muss. Während dieser Phasen wird die Energie gespeichert und zu einem geeigneten Zeitpunkt wieder abgegeben, um die erforderliche aktive Leistung, die von dem Nutzer zur Ausführung der Bewegung aufgebracht werden muss, zu minimieren. Die Aktuator-Dämpfer-Einheit ist an der prothetischen oder orthetischen Vorrichtung so anzuordnen, dass zwei relativ zueinander verlagerbare Komponenten der orthetischen oder prothetischen Vorrichtung über die Aktuator-Dämpfer-Einheit miteinander gekoppelt sind. Dabei wird ein Teil oder eine Komponente der Vorrichtung mit der Kolbenstange gekoppelt ist, während bevorzugt das Gehäuse oder aber eine an oder in dem Gehäuse angeordnete Komponente, die nicht der erste Kolben oder die Kolbenstange ist, an der anderen Komponente der Vorrichtung festgelegt oder daran gekoppelt ist. Die Koppelung zwischen der Kolbenstange und der Vorrichtung muss nicht unmittelbar sein, es kann ein Zwischenelement zwischen der Kolbenstange und der Vorrichtung angeordnet sein, das auch weitere Funktionen aufweisen kann. Als weitere Funktion kann das Zwischenelement eine Kraftsensorik oder Drucksensorik aufweisen. Die Festlegung an der Vorrichtung erfolgt, um Energie aus der orthetischen oder prothetischen Vorrichtung in die Aktuator-Dämpfer-Einheit einspeisen und aus ihr abführen zu können.

Die Energie wird bevorzugt in der volumenveränderbaren Fluidkammer gespeichert, beispielsweise dadurch, dass der weitere Kolben über einen komprimierbaren Energiespeicher mit dem ersten Kolben gekoppelt ist. Der komprimierbare Energiespeicher ist bevorzugt in der volumenveränderbaren Fluidkammer angeordnet und kann als Feder, Elastomerelement und/oder kompressibles Fluidvolumen, beispielsweise ein Gaskissen oder Gasvolumen, ausgebildet sein. Der Energiespeicher kann als Zugelement und/oder als Druckelement ausgeführt sein. Die Speicherung der Energie erfolgt dabei durch eine Volumenveränderung in der Kammer, in der der Energiespeicher angeordnet ist, und das anschließende Fixieren oder Beibehalten des Volumens durch Verschließen der Zugangsöffnung. Erst wenn der Energiespeicher die Energie wieder abgeben soll, wird die Fluidkammer geöffnet, der Speicher entspannt sich und einer Volumenänderung der Fluidkammer tritt ein. Ist beispielsweise eine Feder auf Zug belastet, versucht sie das Volumen der Fluidkammer zu verringern, was bei einem geschlossenen Ventil nicht gelingt. Wird das Ventil geöffnet, zieht sich die Feder zusammen, das Volumen der Kammer verringert sich und die Kolben werden in die entsprechende Richtung bewegt. Neben der Integration des Energiespeichers in den Zylinder ist es auch möglich, die Kopplung über den komprimierbaren Energiespeicher außerhalb des Zylinders durchzuführen, beispielsweise in einem Druck beaufschlagbaren Ausgleichsbehälter oder einem Druckspeicher, der federbelastet ist. Sofern kein Energiespeicher in der Aktuator-Dämpfer-Einheit vorhanden ist oder funktional aktiviert wird, kann die Aktuator-Dämpfer-Einheit als reiner Dämpfer mit einer vergrößerten Variabilität der Dämpfereigenschaften aufgrund der weiteren, volumenveränderbaren Fluidkammer arbeiten. Die Aktuator-Dämpfer-Einheit kann somit auch als reine Dämpfer-Einheit eingesetzt werden.

Jede Fluidkammer kann zumindest eine Zugangsöffnung aufweisen und über Fluidleitungen und/oder Ventile mit zumindest einer anderen Fluidkammer verbunden sein. Über die Fluidleitungen kann ein Volumenausgleich der sich volumenverändernden Kammern, beispielsweise einer Extensionskammer oder einer Flexionskammer sowie der zusätzlichen, volumenveränderbaren Fluidkammer, erfolgen. Durch die Ausbildung beispielsweise eines Überströmkanals von einer Flexionskammer in eine Extensionskammer in der Kolben-Zylinder-Einheit kann das Fluid in die sich jeweils vergrößernde Fluidkammer einströmen. Bei einer Flexion verringert sich das Volumen der Flexionskammer, korrespondierend vergrößert sich das Volumen der Extensionskammer, so dass das aus der Flexionskammer herausgedrückte Volumen ganz oder teilweise in die Extensionskammer überströmen kann.

Jeder Fluidkammer kann zumindest ein Ventil zugeordnet sein, über das der Fluidstrom in und aus der Fluidkammer einstellbar ist. Über die Ventile ist es auch möglich, den Zugang vollständig zu sperren oder auch Fluidleitungen miteinander zu verbinden oder voneinander zu trennen, so dass verschiedene Kombinationen von Überströmleitungen und Verschaltungen von Fluidkammern erzielbar sind. Die Ventile können als Schaltventil, Stellventil oder Rückschlagventil ausgebildet sein, um die gewünschten Fluidströme und Widerstände innerhalb der Fluidströme bereitstellen zu können. Über Stellventile oder Regelventile kann der Strömungsquerschnitt verändert werden, entweder in diskreten Schritten oder kontinuierlich. Rückschlagventile sperren eine Fluidleitung in eine Strömungsrichtung und geben diese in die entgegengesetzte Strömungsrichtung frei, ohne dass ein weiterer Schalt- oder Regelungsaufwand notwendig wäre. Über ein Schaltventil, beispielsweise ein 3-Wege-Ventil, können verschiedene Fluidleitungen miteinander verschaltet werden, um für die jeweilige Bewegung das benötigte Maß an Dämpfung oder aber Energiezufuhr bereitzustellen und Kammer miteinander oder mit weiteren Einrichtungen zu verbinden oder von diesen zu trennen. Rückschlagventile können sicherstellen, dass der Energiespeicher nur bei einem ausreichenden Arbeitsdruck, der höher als der Speicherdruck ist, geladen wird. Mit einem Rückschlagventil kann eine "Ladeleitung" realisiert werden, bei der unbeabsichtigtes Entladen bei zu geringem Arbeitsdruck verhindert wird. Wird das Speichervolumen über zwei getrennte Leitungen angeschlossen, die mit jeweils gegenseitig orientierten Rückschlagventilen versehen sind, so erhält man definierte Leitungen für das Laden und Entladen. Dies kann für die Ventilsteuerung vorteilhaft sein. Will man ein reines Federverhalten realisieren, so ist es ausreichend, das Speichervolumen mit einer Leitung ohne Rückschlagventile zu verbinden. Ebenso besteht die Möglichkeit, die Viskosität des Fluides zu beeinflussen, um die Dämpfung zu verändern, beispielsweise über magnetorheologische Fluide und einstellbare Magnetfelder.

Zumindest ein Ausgleichsvolumen kann mit zumindest einer der Fluidkammern gekoppelt sein, um insbesondere Volumenschwankungen der dritten Fluidkammer oder der weiteren Fluidkammer ausgleichen zu können. Das Ausgleichsvolumen kann außerhalb des Zylinders angeordnet sein, alternativ ist das Ausgleichsvolumen in dem Zylinder integriert. Auch können beispielsweise Volumenunterschiede aufgrund der Kolbenstange oder temperaturbedingter Ausdehnungen des Fluides durch das Ausgleichsvolumen ausgeglichen werden. Das Ausgleichsvolumen kann über zumindest ein Ventil mit zumindest einer der Fluidkammern gekoppelt sein. Ebenso kann das Ausgleichsvolumen als Druckspeicher ausgebildet sein, also beispielsweise ein kompressibles Medium, eine Feder oder einen anderen mechanischen Energiespeicher aufweisen, gegen den das Ausgleichsvolumen mit dem Fluid befüllt werden muss. Zur Abgabe des Fluids wird der Druckspeicher entspannt. Über den Druckspeicher kann auch ein Vordruck im System realisiert werden. Dies ist zur Verringerung einer Kavitationsneigung und zur Verringerung einer Geräuschbildung vorteilhaft.

Als zusätzliche Komponente kann eine Pumpe zur Erhöhung des Fluiddruckes der Aktuator-Dämpfer-Einheit zugeordnet sein, um für den Fall der fehlenden Leistung die Möglichkeit zu geben, zusätzlich Energie in das Fluidsystem einzuspeichern.

Dazu kann die Pumpe mit einem externen Energiespeicher, insbesondere einem Akkumulator oder einer Batterie gekoppelt sein, wobei die Pumpe dann über einen Elektromotor angetrieben wird. Über die Pumpenleistung kann der Speicher geladen werden, um aus dem Speicher die benötigte Energie abrufen zu können. Ist das Energieniveau in dem Speicher nicht hoch genug, wird über die Pumpe eine Niveauanhebung bewirkt. Über die Pumpe kann das Gelenk auch direkt, ohne Zwischenschaltung des Speichers aktiv bewegt werden, wobei sowohl für das Aufladen des Speichers als auch für die direkte Bewegung die Pumpe vorteilhafterweise von dem Steuergerät aus gesteuert wird.

Eine Weiterbildung der Erfindung sieht vor, dass die Pumpe nicht der ersten Kolben-Zylinder-Einheit direkt zugeordnet ist, sondern einer zweiten Kolben-Zylinder-Einheit, die gleichgerichtet zu der ersten Kolben-Zylinder-Einheit geschaltet ist. Die zweite Kolben-Zylinder-Einheit kann parallel zu der ersten Kolben-Zylinder-Einheit anageordnet sein, gegebenenfalls an den gleichen Befestigungspunkten wie die erste Kolben-Zylinder-Einheit, so dass eine Parallelschaltung der beiden Kolben-Zylinder-Einheiten vorliegt. Alternativ kann die zweite Kolben-Zylinder-Einheit seriell zu der ersten Kolben-Zylinder-Einheit geschaltet sein, was die Länge der Aktuator-Dämpfer-Einheit vergrößert. Durch die zweite Kolben-Zylinder-Einheit wird die über die Pumpe aufgebrachte Kraft zusätzlich zu der in der ersten Kolben-Zylinder-Einheit gespeicherten Energie abgegeben.

Bevorzugt ist das Fluid als Hydraulikfluid ausgebildet, so dass eine hydraulische Aktuator-Dämpfer-Einheit vorliegt. Pneumatische Komponenten können beispielsweise als kompressibles Druckmedium und Energiespeicher vorgesehen sein.

Eine Steuereinrichtung kann den Ventilen zu deren Verstellung oder Schaltung zugeordnet sein, um eine elektrisch oder elektronisch gesteuerte Betätigung der Ventile und damit die Steuerung des Fluidstromes zu ermöglichen. Die Steuereinrichtung kann mit Sensoren gekoppelt sein, beispielsweise Winkelsensoren, Kraftsensoren, Kolbenpositionssensoren und/oder Drucksensoren, die Zustandsdaten der Aktuator-Dämpfer-Einheit und/oder der orthetischen oder prothetischen Vorrichtung der Steuereinrichtung übermitteln. Auf Grundlage dieser Sensordaten und von Daten von Sensoren, die Aufschluss über den Bewegungszustand der orthetischen oder prothetischen Einrichtung am Patienten geben, wie zum Beispiel Gyroskope und/oder Beschleunigungssensoren, kann dann über die Steuereinrichtung eine Schaltung der Ventile und eine Regelung der jeweiligen Durchflussmengen durchgeführt und eine Entscheidung darüber getroffen werden, ob und wie gespeicherte Energie abgegeben, ein reiner Dämpferbetrieb durchgeführt oder Energie gespeichert werden soll. Die Steuereinrichtung arbeitet insbesondere elektronisch und verarbeitet elektrische, optische oder anderweitige Sensorsignale mittels einer Datenverarbeitungseinrichtung, einem Prozessor oder einem Computer. Die Steuereinheit veranlasst die Verstellung von Ventilen oder Magnetfeldern, um Widerstände zu verändern oder Strömungswege zu öffnen oder zu schließen.

Eine Weiterbildung der Erfindung sieht vor, dass zwei weitere Kolben in dem Zylinder angeordnet sind, die zwei weitere, volumenveränderbare Fluidkammern ausbilden. Dadurch ist es möglich, insgesamt vier Fluidkammern bereitzustellen, wodurch sowohl das Speichern als auch das Abgeben in beiden Bewegungsrichtungen erfolgen kann. Eine kompakte Bauweise wird erzielt, wenn die beiden weiteren Kolben auf einander gegenüberliegenden Seiten des ersten Kolbens angeordnet sind. Als Alternative ist vorgesehen, dass die beiden weiteren Kolben in von dem ersten Zylinder strömungstechnisch entkoppelten Zylinder angeordnet sind, die jedoch mechanisch miteinander verbunden sind. Beispielsweise kann ein erster weiterer Kolben in einer getrennten Kammer des ersten Zylinders unter Ausbildung zweier Fluidkammern elastisch gelagert sein, während die Kolbenstange des ersten Kolbens an einem Gehäuse eines zweiten, separaten, verlagerbaren Gehäuses angeordnet ist, indem ein zweiter, weiterer, also dritter Kolben elastisch gelagert ist, der wiederum unmittelbar über eine Kolbenstange an der Vorrichtung gelagert ist. Somit sind drei Kolben-Zylinder-Einheiten in Serie geschaltet, die sechs Fluidkammern ausbilden, wobei in zwei Fluidkammern eine Energiespeicherung stattfinden kann. Dadurch wird ein Speichern und Abgeben von Energie in beiden Bewegungsrichtungen ermöglicht.

Nachfolgend wird die Erfindung anhand der beigefügten Figuren näher erläutert. Gleiche Bezugszeichen bezeichnen gleiche Komponenten. Es zeigen:
- Figur 1: eine schematische Darstellung einer Aktuator-Dämpfer-Einheit;
- Figur 2: eine Variante der Figur 1 im montierten Zustand;
- Figur 3: eine Variante der Figur 2 ohne Pumpe;
- Figur 4: ein hydraulisches Schaltbild;
- Figur 5: eine Schnittdarstellung durch eine Aktuator-Dämpfer-Einheit;
- Figuren 6 bis 16: Darstellungen verschiedener Belastungszustände und Ventilstellungen;
- Figur 17: eine weitere Variante der Erfindung;
- Figur 18: ein alternatives 3-Wege-Ventil;
- Figur 19: eine schematische Darstellung einer Variante mit zwei Zusatzkolben;
- Figur 20: eine schematische Darstellung einer Variante mit einer zweiten Kolben-Zylinder-Einheit und zwei Zylindergehäusen;
- Figur 21: eine Variante der Figur 20 mit vier Fluidkammern und einer zusätzlichen Kolben-Zylinder-Einheit;
- Figur 22: eine Schnittdarstellung einer Aktuator-Dämpfer-Einheit mit vier Fluidkammern;
- Figur 23: eine Variante der Erfindung mit einem separaten, beweglichen Kolben;
- Figur 24: eine Variante der Figur 23 mit einer Pumpe;
- Figuren 25 bis 29b: Anwendungsbeispiele der Aktuator-Dämpfereinheit;
- Figur 30: eine schematische Darstellung eines Kolbens mit einstellbarem Energiespeicher;
- Figur 30b: eine Variante der Figur 30;
- Figur 31: eine Schnittdarstellung einer Aktuator-Dämpfereinheit mit zwei Kolbenstangen;
- Figur 32: eine Schnittdarstellung eines mechanischen Druckregelventils;
- Figur 33: eine Einbausituation des Druckregelventils in schematischer Darstellung;
- Figur 34 bis 39: schematische Schnittdarstellungen einer Variante mit drei Kolben in einem durch zwei Sperrwände in drei hydraulisch miteinander verbundenen Kammern unterteilten Zylindern.

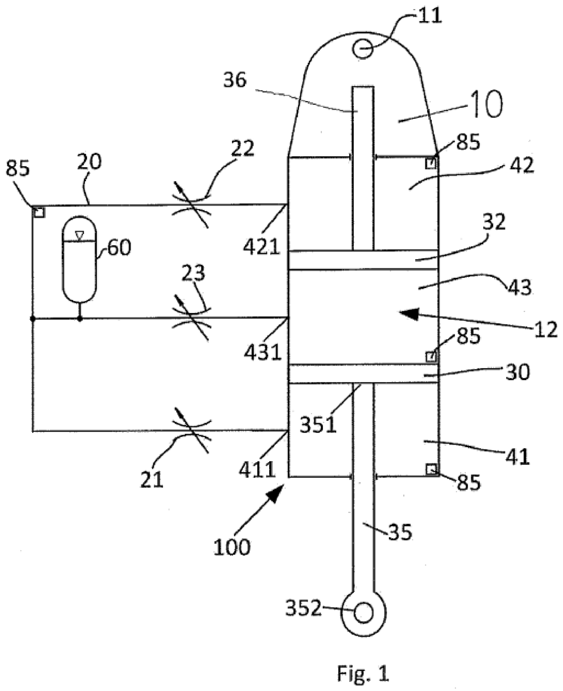

Figur 1 zeigt in einer schematischen Darstellung eine Aktuator-Dämpfer-Einheit 100 zum Einsatz in einer prothetischen oder orthetischen Vorrichtung, beispielsweise in einer Prothese oder Orthese. Die Aktuator-Dämpfer-Einheit, nachfolgend AD-Einheit, weist ein Gehäuse 10 auf, in dem ein Zylinder 12 ausgebildet ist. Im dargestellten Ausführungsbeispiel weist der Zylinder 12 einen kreisförmigen Querschnitt auf und nimmt in sich einen ersten Kolben 30 auf, der entlang der Zylinderwand verfahrbar gelagert ist. Die Form des Kolbens kann auch abweichend ausgebildet sein und kenn einen nicht kreisförmigen Querschnitt aufweisen. An dem ersten Kolben 30 ist eine Kolbenstange 35 angeordnet, deren erstes Ende 351 mit dem ersten Kolben 30 verbunden ist. Das zweite, dem ersten Ende 351 gegenüberliegende Ende 352 der Kolbenstange 35 ist an der prothetischen oder orthetischen Vorrichtung festlegbar. Das Gehäuse 10 weist eine Befestigungseinrichtung 11 auf, über die das Gehäuse 10 an einer anderen Prothesenkomponente oder Orthesenkomponente festlegbar ist. Werden die beiden Orthesen- oder Prothesenkomponenten relativ zueinander verlagert, wird der Kolben 30 innerhalb des Zylinders 12 bewegt, so dass sich eine Volumenveränderung einer ersten Fluidkammer 41 einstellt. Korrespondierend zu einer Volumenverringerung der ersten Fluidkammer 41 vergrößert sich das gegenüberliegende Volumen des Zylinders 12, das durch den ersten Kolben 30 unterteilt wird. In diesem zweiten Volumen ist ein zweiter Kolben 32 entlang der Längserstreckung des Zylinders 12 verschieblich angeordnet. Der zweite Kolben 32 unterteilt die zweite Fluidkammer 42, so dass sich zwischen den beiden Kolben 30, 32 eine dritte, volumenveränderbare Fluidkammer 43 ausbildet. Innerhalb jeder Fluidkammer 41, 42, 43 kann ein Sensor 85 in Gestalt eines Drucksensors angeordnet sein, um den in der jeweiligen Fluidkammer 41, 42, 43 vorherrschenden Druck erfassen zu können. In einer anderen Ausführungsform können andere Sensoren vorgesehen sein. Die Sensordaten der Sensoren 85 werden an eine Steuereinrichtung übermittelt, die später näher erläutert werden wird.

An dem zweiten Kolben 32 ist eine weitere Stange 36 angeordnet, die aus dem Gehäuse 10 herausführt. Weist die zweite Stange 36 den gleichen Querschnitt wie die Kolbenstange 35 auf, so muss bei einer reinen Verlagerung der beiden Kolben 30, 32 ohne Volumenveränderung der mittleren Fluidkammer 43 kein Ausgleichsvolumen für das transportierte Fluid bereitgestellt werden. Ein solches Ausgleichsvolumen ist notwendig, wenn nur eine Kolbenstange 35 vorhanden ist und das Fluid, das bevorzugt und im dargestellten Ausführungsbeispiel als Hydraulikfluid ausgebildet ist, im Wesentlichen inkompressibel ist. Verzichtet man auf die weitere Stange 36, muss das von der Kolbenstange verdrängte Volumen ausgeglichen werden, z.B. über ein Ausgleichsvolumen.

Jede der Fluidkammern 41, 42, 43 ist mit einer Zugangsöffnung 411, 421, 431 versehen, über die das Hydraulikfluid aus der jeweiligen Fluidkammer 41, 42, 43 aus- und wieder hineinströmen kann. Die Zugangsöffnungen 411, 421, 431 sind über Fluidleitungen 20 miteinander verbunden. Vor jeder Zugangsöffnung 411, 421, 431 ist ein Schalt- oder Stellventil 21, 22, 23 in der Fluidleitung 20 angeordnet, um den Strömungsquerschnitt der Fluidleitung 20 und damit auch den hydraulischen Widerstand einstellen zu können.

Innerhalb der weiteren, volumenveränderbaren Fluidkammer 43 kann ein kompressibles Medium oder eine Feder angeordnet sein, so dass bei einem geschlossenen Ventil 22 und einem zumindest teilweise geöffneten Ventil 23 das Volumen der dritten Fluidkammer 43 verringert wird. Dadurch wird das kompressible Medium komprimiert und Energie gespeichert. Durch die Volumenveränderung innerhalb der Fluidkammer 43 ist es notwendig, dass ein Ausgleichsvolumen 60 der AD-Einheit zugeordnet ist, so dass ein Volumenausgleich, z.B. aufgrund einer Leckage, einer einfahrenden Kolbenstange oder Temperaturschwankungen, stattfinden kann. Über einen optionalen Drucksensor 85 kann der Druck in dem Ausgleichsvolumen 60 gemessen werden, dies gibt Aufschluss über die Verringerung des Vordruckes aufgrund von Fluidverlusten. Wird das Ventil 23 geschlossen, verhält sich die dritte Fluidkammer 43 quasi starr, so dass eine normale Dämpferhydraulik bereitgestellt werden kann, beispielsweise mit einer Flexionskammer 41 und einer Extensionskammer 42. Sobald das Ventil 23 wieder geöffnet wird, entspannt sich das komprimierte Medium beziehungsweise die Feder oder das elastische Element und der Kolben 30 wird entgegen der Kompressionsrichtung nach außen gedrückt, wodurch eine entsprechende Bewegung in der orthetischen oder prothetischen Vorrichtung bewirkt oder unterstützt wird.

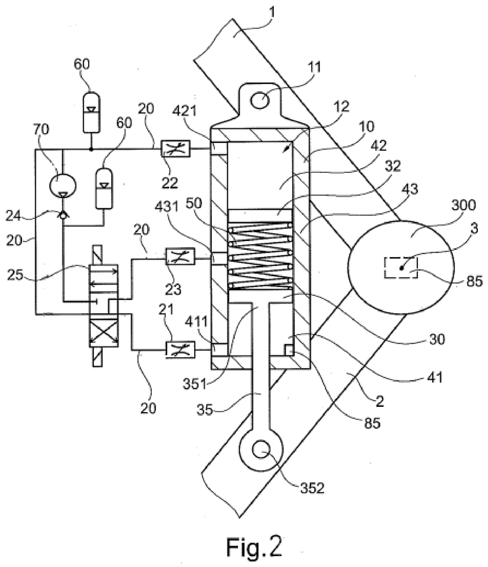

Eine Variante der Erfindung ist in der Figur 2 dargestellt, bei der die AD-Einheit mit dem Gehäuse 10 an einem Oberteil 1 einer orthetischen oder prothetischen Gelenkeinrichtung über ein Befestigungselement 11, beispielsweise einen Bolzen, befestigt ist. An dem Oberteil 1 ist ein Unterteil 2 drehbar um eine Schwenkachse 3 gelagert, die in einer Gelenkeinrichtung 300 ausgebildet ist. Die Gelenkeinrichtung verbindet das Oberteil 1 mit dem Unterteil 2 und kann als eine einfache Schwenkachse 3 oder als eine Polyzentrik ausgebildet sein. Die Kolbenstange 35 des Kolbens 30 ist mit dem zweiten Ende 352 schwenkbar an dem Unterteil 2 festgelegt. Wird der Kolben 30 nach unten bewegt, extendiert die Gelenkeinrichtung, wird der Kolben 30 nach oben bewegt, verringert sich der Gelenkwinkel, die Gelenkeinrichtung flektiert. Um die Gelenkachse 3 herum kann ein Sensor 85 zur Erfassung der Relativposition, z.B. eines Winkels, zwischen dem Oberteil 1 und dem Unterteil 2 angeordnet sein, über den Positionsdaten, z.B. Winkeldaten an eine Steuerungseinrichtung 80 übermittelt werden. Bei einem polyzentrischen Gelenk erfolgt ebenfalls eine sensorbasierte Winkelmessung, die jedoch auch über mehrere Sensoren 85 erfolgen kann. Die Sensoren 85 in Gestalt von Drucksensoren innerhalb der AD-Einheit sind nur angedeutet und liefern ebenfalls Steuersignale für die Steuereinheit 80, die nur angedeutet ist und einen Ventilblock ansteuert, der Teil der Steuereinheit 80 sein kann. Alternativ oder ergänzend kann auch die Position des Kolbens 30 und die von der AD-Einheit aufgebrachte Kraft gemessen und der Steuerungseinrichtung 80 übermittelt werden.

Der mechanische und hydraulische Aufbau der Ausgestaltungsform gemäß Figur 2 entspricht im Wesentlichen dem der Figur 1, auch hier sind drei Zugangsöffnungen 411, 421, 431 vorhanden, denen jeweils ein Stellventil 21, 22, 23 zugeordnet ist. Das jeweilige Stellventil 21, 22, 23 wird aufgrund von Steuersignalen der Steuereinheit 80 per Verstelleinrichtung verstellt, also der Strömungsquerschnitt der Fluidleitung 20 vergrößert, verkleinert oder der Durchfluss gesperrt. Im dargestellten Ausführungsbeispiel ist das Ausgleichsvolumen 60 mit einem Vordruck beaufschlagbar. Darüber hinaus ist zur Bereitstellung zusätzlicher Energie eine Pumpe 70 vorgesehen, die über ein Rückschlagventil 24 in Verbindung mit einem Ausgleichsvolumen 60 oder Speicher und einem 3-Wege-Ventil 25 mit dem hydraulischen System der AD-Einheit gekoppelt ist. Über die Pumpe 70 kann zusätzliche potentielle oder kinetische Energie in das System eingebracht werden, so dass, für den Fall, dass die innerhalb der weiteren, volumenveränderbaren Fluidkammer 43 gespeicherte Energie nicht ausreicht, die gewünschte Bewegung einzuleiten oder auszuführen, zusätzliche Bewegungsenergie bereitgestellt werden kann. Die Pumpe 70 kann zusätzlich zu der gespeicherten mechanischen Energie eingesetzt werden. Ebenso ist es möglich, über die Pumpe 70 Energie in das hydraulische System einzuspeisen, so dass ein Energiespeicher 50, der im dargestellten Ausführungsbeispiel als Wendelfeder ausgebildet ist, aufgeladen werden kann. Der Energiespeicher 50 ist als elastisches Element ausgebildet, dass sowohl als Druckelement als auch als Zugelement ausgeführt sein kann. Die Ausgestaltung des Energiespeichers als elastisches Element ist nicht auf die konkrete Ausführungsform beschränkt.

In der dargestellten Schaltstellung des 3-Wege-Ventils 25 ist die Pumpe 70 abgekoppelt. Wird das Ventil 25 nach unten bewegt, entsteht eine Verbindung zwischen der Pumpe 70 und der dritten, weiteren Fluidkammer 43, so dass hier ein Druckaufbau innerhalb der Kammer 43 stattfinden kann, wenn das vorgeschaltete Stellventil 23 geöffnet ist. Die beiden anderen Fluidkammern 41, 42 sind dann über die Fluidleitung 20 und die Ventile 21, 22 miteinander verbunden. Falls durch den beaufschlagten Druck über die Pumpe 70 eine Volumenvergrößerung innerhalb der Kammer 43 stattfindet, wird diese durch das Ausgleichsvolumen 60 aufgefangen.

Wird das 3-Wege-Ventil nach oben verfahren, wird die erste Fluidkammer 41 über die Pumpe 70 mit hydraulischem Druck beaufschlagt, so dass das Volumen der dritten Fluidkammer 43 verringert wird, wodurch bei geöffnetem Ventil 23 die Feder 50 als Energiespeicher komprimiert wird.

Ist das dritte Ventil 23 der zwischen den Kolben 30, 32 gebildeten Fluidkammer 43 gesperrt, und sind die beiden anderen Ventile 23, 22 der endseitigen Fluidkammern 41, 42 offen, ist die AD-Einheit frei beweglich. Die Energie in dem Speicher wird dissipiert, wenn das Ventil 21 der ersten Ventilkammer gesperrt ist und die beiden anderen Ventile 22, 23 geöffnet sind. Energie wird gespeichert oder abgegeben, wenn das Ventil 22 der oberen Fluidkammer 42 geschlossen ist und die beiden anderen Ventile 21, 23 geöffnet sind. Ist das obere Ventil 22 geöffnet und das Ventil 23 der zentralen Fluidkammer 43 stärker gedrosselt als das Ventil 21 der ersten Fluidkammer 41, wird Energie abgegeben, jedoch in einer der Speicherbewegung entgegengesetzten Bewegungsrichtung.

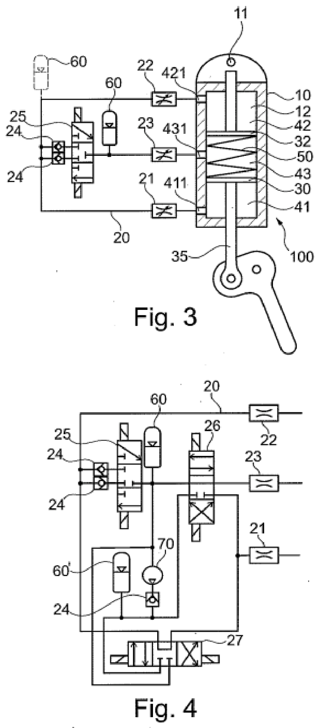

Figur 3 zeigt eine weitere Variante der Erfindung mit einem grundsätzlich ähnlichen hydraulischen Aufbau. Statt einer Pumpe 70, wie in der Figur 2 dargestellt, die über einen Elektromotor angetrieben werden kann, sieht die AD-Einheit gemäß Figur 3 eine rein mechanische Energiespeicherung vor, die in dem Energiespeicher 50 in Gestalt der Feder in der eingeschlossenen Fluidkammer 43 realisiert wird. Die Feder 50 verbindet die beiden Kolben 30, 32 miteinander und koppelt diese krafttechnisch. Die Kolbenstange 35 ist mit einem Hebel gekoppelt, so dass bei einer Extension, also bei einem Herausbewegen der Kolbenstange 35 aus dem Zylinder 12, der Hebel entgegen dem Uhrzeigersinn verschwenkt wird. Bei einer Flexion, also bei einem Einschieben der Kolbenstange 35 in den Zylinder 12, verdreht der Hebel in Uhrzeigersinn, eine alternative Anordnung des Ausgleichsvolumens ist hier gestrichelt dargestellt. Ein optionales Ausgleichsvolumen ist gestrichelt dargestellt, das Ausgleichsvolumen 60 kann mit einem Vordruck belastet sein.

Dem 3-Wege-Ventil 25 sind zwei Rückschlagventile 24 zugeordnet, um den Zustrom zu der zusätzlichen Fluidkammer 43 bzw. von der zusätzlichen Fluidkammer 43 zu den beiden außenliegenden Fluidkammern 41, 42 zu ermöglichen oder zu sperren. In der dargestellten Schaltstellung des 3-Wege-Ventils 25 findet kein Volumenaustausch zwischen den beiden außenliegenden Fluidkammern 41, 42 und dem Fluidvolumen in der dritten Fluidkammer 43 statt. Wird die Feder 50 komprimiert, wenn das dritten Ventil 23 geöffnet wird, wird das Fluid in den Ausgleichsbehälter 60 verlagert. Das 3-Wege-Ventil 25 sieht einmal eine Abkopplung der mittleren Fluidkammer 43 vor, einmal wird ein Einlass von den beiden umgebenden Fluidkammern 41, 42 in die mittlere, eingeschlossene Fluidkammer 43 ermöglicht, nämlich, wenn das 3-Wege-Ventil 25 in der unteren Stellung ist, so dass von dem oberen Rückschlagventil ein Zustrom in die Kammer 43 ermöglicht wird. Schließlich ist in der umgekehrten Stellung nur ein Rückstrom des Fluids aus der zusätzlichen Kammer 43 zurück zu den beiden umgebenden Kammern 41, 42 möglich. Wohin und wieviel des Volumens aus der eingeschlossenen Kammer 43 in die jeweiligen umgebenen Kammern 41, 42 strömt, wird über die Stellung der jeweiligen Stellventile 21, 22 festgelegt.

In der Figur 4 ist eine Weiterbildung der Erfindung dargestellt, bei der die Kolben-Zylinder-Einheit 100 nicht mehr dargestellt ist. Der mechanische Aufbau entspricht der der Figur 3. Auch hier sind die jeweiligen Stellventile 21, 22, 23 an den Zugangsöffnungen 411, 421, 431 vorgesehen. Die Schaltung mit dem 3-Wege-Ventil 25 und den beiden vorgeschalteten, gegenläufig orientierten Rückschlagventilen 24 sowie dem Ausgleichsvolumen 60 entspricht der Schaltung gemäß Figur 3. Zusätzlich ist ein weiteres 3-Wege-Ventil 26 zwischen den ersten 3-Wege-Ventil 25 und dem Stellventil 23 der eingeschlossenen Fluidkammer 43 angeordnet. Das zweite 3-Wege-Ventil 26 koppelt oder sperrt den hydraulischen Weg zu der ersten Kammer 41 und sieht in der dargestellten mittleren Stellung einen Durchgang sowie eine hydraulische Trennung zwischen der ersten Fluidkammer 41 und der dritten Fluidkammer 43 vor. Weiterhin ist eine Pumpe 70 vorgesehen, die über ein Rückschlagventil 24 und ein optionales Puffervolumen 60' in Gestalt eines zusätzlichen Druckspeichers mit einem dritten 3-Wege-Ventil 27 gekoppelt ist. Das dritte 3-Wege-Ventil 27 ist in einer hydraulischen Leitung der ersten Fluidkammer 41 eingekoppelt und stellt in der dargestellten mittleren Position eine Durchleitung zur Verfügung. Die dargestellte Schaltstellung entspricht der hydraulischen Anordnung gemäß der Figur 3. Das zweite 3-Wege-Ventil 26 schließt die Fluidleitung 20 zwischen der Pumpe 70 und der ersten Fluidkammer 41, in der oberen Stellung wird eine Verbindung zwischen der Pumpe 70 und der eingeschlossenen, zusätzlichen Fluidkammer 43 durch eine Überkreuzschaltung ermöglicht.

Das dritte 3-Wege-Ventil 27 verbindet die Pumpe 70 mit der oberen, zweiten Fluidkammer 42, in der rechten Stellung erfolgt eine Verbindung mit der ersten Fluidkammer 41.

Mit einer solchen Ventilanordnung lassen sich vielfältige Dämpfungs-, Speicherungs- und Aktuierungsmöglichkeiten realisieren. Zur Speicherung von Energie bei einer Flexionsbewegung ist das obere Ventil 22 geschlossen, das mittlere Ventil 23 gedrosselt und das untere Ventil 21 geöffnet, gleichzeitig ist das erste 3-Wege-Ventil 25 in die rechte Stellung, also nach oben, verschoben. Die beiden anderen 3-Wege-Ventile 26, 27 bleiben in der jeweils mittleren Stellung. Dadurch kann das Volumen der dritten Kammer 43 verringert werden, die Feder 50 wird gespannt und kinetische Energie wird in potentielle Energie umgewandelt und gespeichert.

Zur Abgabe von Energie in der Extensionsrichtung wird das obere Ventil 22 geschlossen gelassen, das mittlere Ventil 23 wird geöffnet und das untere Ventil 21 gedrosselt. Das erste der 3-Wege-Ventile 25 wird in die linke Stellung nach unten verfahren, so dass Fluid aus der ersten Fluidkammer 41 und gegebenenfalls dem Ausgleichsvolumen 60 oder dem optionalen Pufferspeicher 60' in die sich vergrößernde dritte Fluidkammer 43 einströmen kann.

Zum Dämpfen der Flexion wird das obere Ventil 22 gedrosselt, das mittlere Ventil 23 wird geschlossen und das untere Ventil 21 geöffnet. Die 3-Wege-Ventile 25, 26, 27 befinden sich in der dargestellten mittleren Stellung. In dieser Stellung wird der Volumenstrom aus der oberen, zweiten Kammer 42 in die untere, erste Kammer 41 geleitet, der Fluidstrom wird über das obere Drosselventil 22 behindert, die übrigen Komponenten sind nicht beteiligt, es liegt ein reiner Hydraulikdämpfer vor.

Zur Dämpfung in der Extensionsbewegung, bei der die Kolbenstange 35 aus dem Gehäuse 10 herausfährt, wird das obere Ventil 22 geöffnet, das mittlere Ventil 23 bleibt geschlossen und das untere Ventil 21 wird gedrosselt, so dass eine Umkehrung zur der Dämpfung in der Flexion bewirkt wird.

Zum Einbringen einer aktiven Kraft in Flexionsrichtung, also um den ersten Kolben 30 in das Gehäuse 10 hineinzuschieben, wird das obere Ventil 22 geöffnet, das mittlere Ventil 23 geschlossen und das untere Ventil 21 gedrosselt. Die 3-Wege-Ventile 25, 26 befinden sich in der mittleren, dargestellten Stellung, das dritte 3-Wege-Ventil ist nach rechts verschoben, so dass sich eine Überkreuzkopplung der Fluidleitungen ergibt, um die Pumpe 70 mit der unteren, ersten Fluidkammer 41 zu koppeln, so dass diese mit Druck beaufschlagt wird. Dadurch wird eine Flexion aktiv unterstützt.

Umgekehrt wird zu einer aktiven Extension der Druck aus der Pumpe 70 in die obere Kammer 42 geleitet, die auf der der Kolbenstange 35 abgewandten Seite des Kolbens 30 befindlich ist. Das obere Ventil 22 wird gedrosselt, das mittlere Ventil 23 ist geschlossen und das untere Ventil 21 ist offen. Das dritte 3-Wege-Ventil 27 befindet sich in der linken Stellung, so dass die Pumpe 70 durch die Parallelleitung mit der oberen Fluidkammer 42 gekoppelt ist. Aufgrund des geschlossenen mittleren Ventils 23 ergibt sich keine Volumenveränderung in der dritten Kammer 43, so dass sowohl der zweite Kolben 32 als auch der erste Kolben 30 nach unten bewegt wird, so dass eine Extensionsbewegung aktiv über die Pumpe unterstützt wird.

Um neben einer aktiven Energiezufuhr über die Pumpe 70 in Extensionsrichtung auch eine Abgabe mechanisch gespeicherter Energie aus dem Energiespeicher 50 zu bewirken, ist das obere Ventil 22 geschlossen, das mittlere Ventil 23 ist gedrosselt und das untere Ventil ist geöffnet. Das erste 3-Wege-Ventil 25 befindet sich in der Mittelstellung, ebenso wie das dritte 3-Wege-Ventil 27. Das zweite 3-Wege-Ventil 26 befindet sich in der rechten Stellung, so dass zusätzlicher Druck von der Pumpe 70 auf die mittlere Fluidkammer 43 ausgeübt wird, wodurch sich die Feder 50 entspannt und diese Entspannung durch die Pumpe 70 verstärkt wird. Dadurch wird der erste Kolben 30 nach unten bewegt und eine Extensionsbewegung bewirkt.

Eine aktive Beeinflussung der Flexion bei gleichzeitigem Speichern von Flexionsenergie wird bewirkt, wenn das obere Ventil 22 geschlossen ist, das mittlere Ventil 23 geöffnet und das untere Ventil 21 gedrosselt wird. Das erste 3-Wege-Ventil 25 befindet sich in der Mittelstellung, ebenso wie das dritte 3-Wege-Ventil 27. Das zweite 3-Wege-Ventil 26 ist in der linken Stellung, so dass das Fluid von der Pumpe 70 in die erste, untere Fluidkammer 41 eingeleitet wird, um so einerseits die Flexion zu unterstützen und andererseits aufgrund der Ventilstellung die Flexionsenergie in dem Energiespeicher 50 zu speichern.

Sofern durch die Pumpe 70 der mechanische Energiespeicher 50 aufgeladen werden soll, ohne dass sich eine Flexion oder Extension einstellt, sind das obere Ventil 22 und das mittlere Ventil 23 geöffnet, das untere Ventil 21 ist geschlossen. Das erste 3-Wege-Ventil 25 unterbricht den Fluidstrom in der mittleren Stellung, das zweite Ventil 26 steht in der mittleren Stellung, das dritte 3-Wege-Ventil 27 stellt in der linken Stellung eine Kopplung zwischen der Pumpe 70 und der oberen Kammer 42 und der mittleren Kammer 43 her. Das zweite 3-Wege-Ventil 26 befindet sich in der mittleren Stellung, so dass von der Pumpe 70 Fluid in die mittlere Kammer 43 gepumpt wird. Der zweite Kolben 32 entfernt sich von dem ersten Kolben 30, das Volumen innerhalb der dritten Fluidkammer 43 vergrößert sich und die Feder 50 wird auf Zug gespannt.

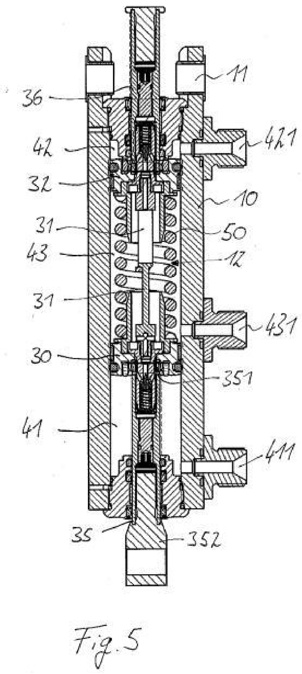

Figur 5 zeigt eine Schnittdarstellung durch eine AD-Einheit ohne Ventile. Das Gehäuse 10 ist im Wesentlichen zylindrisch ausgebildet und weist einen Innenzylinder auf, in dem zwei Kolben 30, 32 längsverschieblich gelagert sind. Der ersten Kolben 30 ist mit einem ersten Ende 351 einer Kolbenstange 35 verbunden, die aus dem Gehäuse 10 herausführt und an dem äußeren, zweiten Ende 352 eine Aufnahme zur Festlegung beispielsweise an einem Prothesenkniegelenk oder einem Orthesenkniegelenk aufweist.

Der zweite Kolben 32 ist ebenfalls mit einer nach außen führenden Stange 36 verbunden und trennt das Zylindervolumen jenseits des ersten Kolbens 30 in zwei Fluidkammern 42, 43. In der mittleren Fluidkammer 43 ist eine Feder 50 als mechanischer Energiespeicher angeordnet. In der dargestellten Position der beiden Kolben 30, 32 sind diese in der maximal oberen Stellung angeordnet, das heißt, dass die erste, untere Fluidkammer 41 das Maximalvolumen aufweist, ebenso weist die zweite, obere Fluidkammer 42 ein Minimalvolumen auf, das Volumen der mittleren Fluidkammer zwischen den beiden beweglichen Kolben 30, 32 ist ebenfalls maximal. Zwischen den beiden Kolben 30, 32 ist eine mechanische Koppeleinrichtung 31 angeordnet, die bei Erreichen des maximalen Volumens der mittleren Kammer 43 in Eingriff tritt und das Maximalvolumen begrenzt. Dadurch ist es möglich, dass durch eine Extensionsbewegung und ein Herausziehen der Kolbenstange 35 aus dem Gehäuse 10 der obere, zweite Kolben 32 über die Koppeleinrichtung 31 mitgenommen wird. Dadurch kann eine Vorspannung der Feder 50 und somit eine Energiespeicherung erreicht werden.

Die jeweiligen Zugangsöffnungen 411, 421, 431 sind an den Endbereichen des Zylinders 12 sowie in der Nähe der Maximalposition des ersten Kolbens 30 angeordnet. Die Feder 50 ist zentral über Dorne geführt, um eine Verkippung oder Verklemmung zu vermeiden. Alternativ zu einer Ausgestaltung mit einer Wendelfeder oder Schraubenfeder kann der Energiespeicher auch als Spiralfeder, Tellerfeder oder Tellerfederpakete, als mechanische Feder jeglicher Art, als pneumatisches Element oder Elastomerbauteil ausgebildet sein, wobei sowohl Zug- als auch Druckfedern zum Einsatz kommen können.

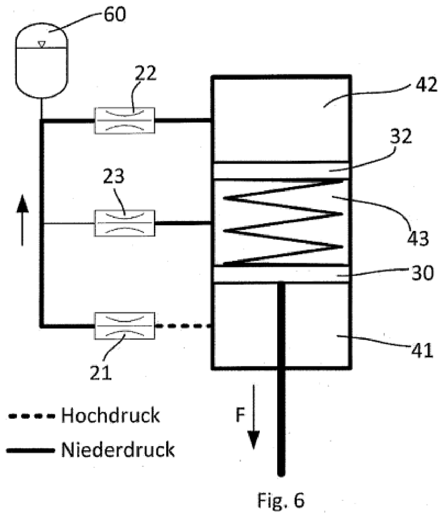

In der Figur 6 ist die Schaltposition bei einer Dämpfung für die Extension dargestellt, das obere Ventil 22 ist geöffnet, das untere Ventil 21 ist gedrosselt und das mittlere Ventil 23 ist geschlossen.

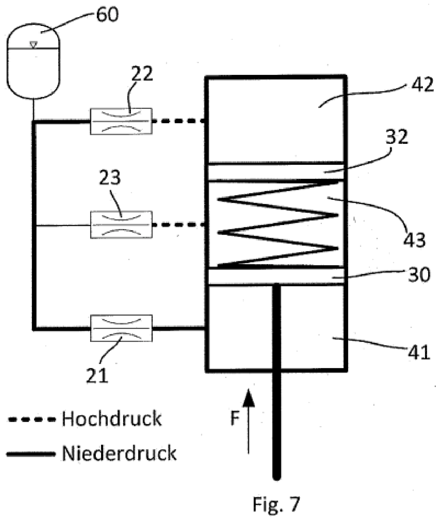

Gemäß Figur 7 wird bei einer Flexion das obere Ventil 22 gedrosselt, das untere Ventil 21 geöffnet und das mittlere Ventil 23 geschlossen, wobei die Feder 50 wirkungslos ist, da aus der Kammer 43 kein Fluidaustausch stattfinden kann, wodurch die Kolben 30 und 32 zu einem virtuellen Starrkörper verbunden werden. Die Wirkung der Kraft F wird über das Ventil 22 kontrolliert dissipiert.

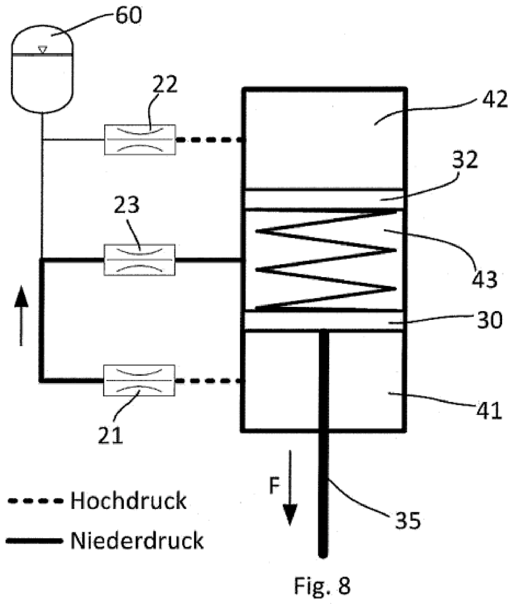

In der Figur 8 wird die Freigabe von Energie aus dem Speicher bei der Extension, also ausfahrender Kolbenstange 35, in Verbindung mit einer Drosselung dargestellt. Das obere Ventil 22 ist geschlossen, das mittlere Ventil 23 ist geöffnet und das untere Ventil 21 ist gedrosselt, wobei in der oberen Kammer 42 ein hoher Druck anliegt und der Fluidstrom von der unteren Kammer 41 gedrosselt in die mittlere Kammer 43 strömt. Das aufgrund des Kolbenstangenvolumens fehlende Fluidvolumen wird aus dem Ausgleichsvolumen 60 ergänzt.

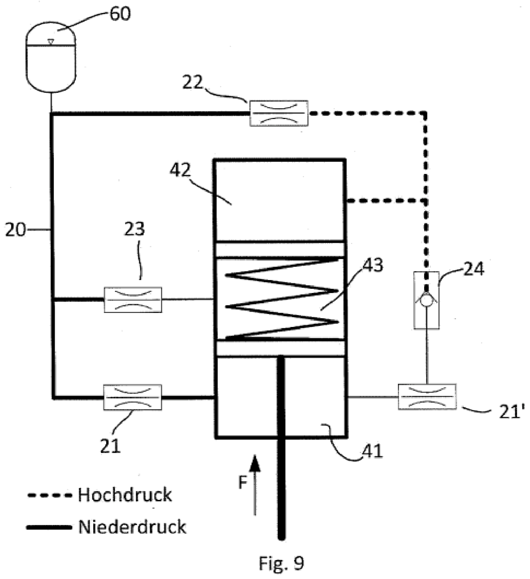

In der Figur 9 ist eine andere Führung der Fluidleitungen dargestellt. Von der ersten Fluidkammer 41 zweigen zwei Fluidleitungen 20 ab, die jeweils über Schalt- oder Stellventile mit den jeweiligen Zugangsöffnungen verbunden sind. Zum Dämpfen in der Flexionsbewegung ist das obere Ventil 22 gedrosselt, das Ventil 23 für die mittlere Fluidkammer 43 ist geschlossen, ebenso wie das rechte Zusatzventil 21', das der ersten unteren Fluidkammer zugeordnet und einem Rückschlagventil 24 vorgeschaltet ist. Das linke Stellventil 21 der unteren Fluidkammer 41 ist geöffnet, so dass bei einer Flexionsbewegung das Fluid aus der oberen Fluidkammer 42 durch das Drosselventil 22 in die untere Fluidkammer 41 einströmen kann, der Drosseleffekt tritt an dem oberen Ventil 22 auf.

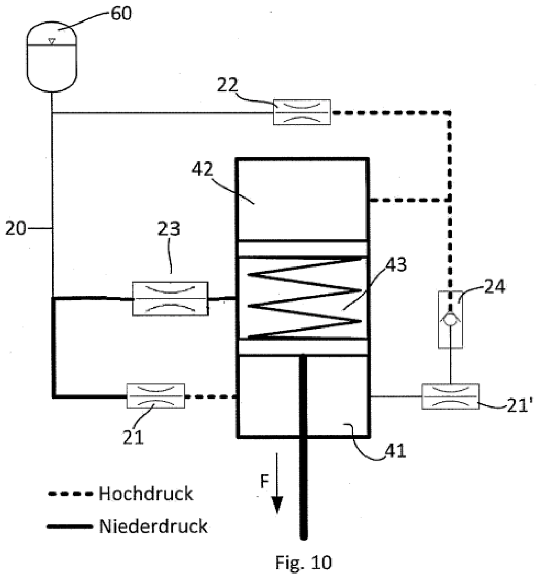

Figur 10 zeigt den gleichen mechanischen Aufbau der AD-Einheit gemäß Figur 9. Zur Abgabe von Energie aus der mittleren Fluidkammer 43 wird das erste Ventil 21 gedrosselt. Weiterhin wird das dritte Ventil 23 zur mittleren Fluidkammer geöffnet, so dass Fluid aus der ersten Fluidkammer 41 in die mittlere Fluidkammer 43 strömen kann. Das zweite Stellventil 22 zur oberen Fluidkammer 42 ist geschlossen.

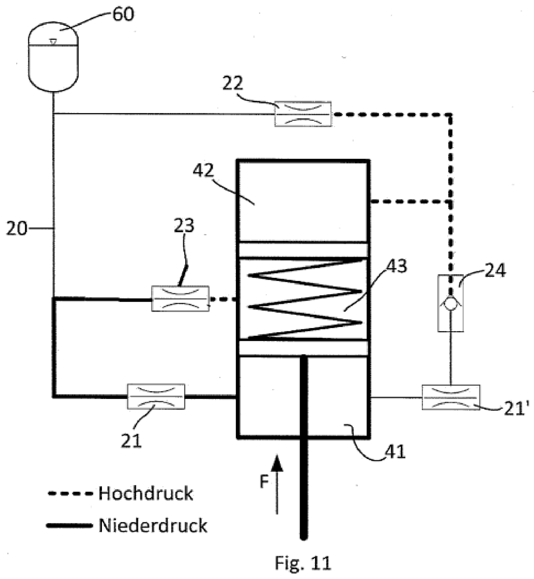

Die gleiche Ventilstellung ergibt sich in der Figur 11, bei der das Speichern von Bewegungsenergie bei einer Flexionsbewegung durchgeführt wird. Das erste Zusatzventil 21'ist geschlossen, ebenso wie das zweite Ventil 22. Das dritte Ventil 23 ist gedrosselt, so dass eine Volumenverringerung der Fluidkammer 43 ermöglicht wird, beispielsweise durch ein weiteres Rückschlagventil 24, das nur eine Volumenverringerung der dritten Fluidkammer 43 zulässt. Anders als bei der Ausgestaltung gemäß Figur 10, bei der das Einströmen durch das vorgeschaltete Rückschlagventil 24 in die dritte Fluidkammer 43 ermöglicht wird, wird hier das Ausströmen ermöglicht.

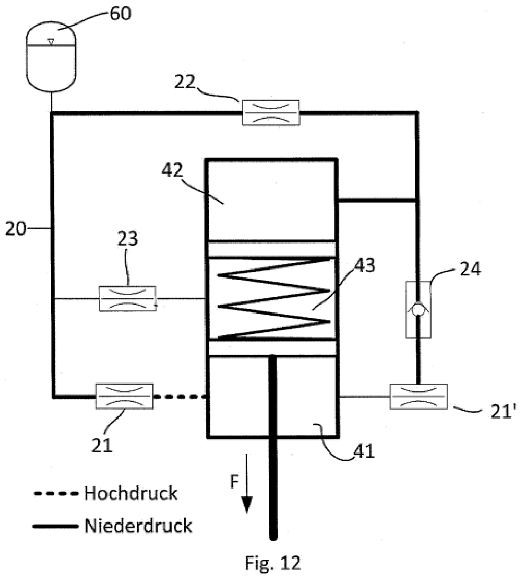

Zum Dämpfen in der Extension gemäß Figur 12 wird das obere Ventil 22 geöffnet, das mittlere Ventil 23 ist geschlossen und das erste Ventil 21 ist gedrosselt, das Zusatzventil 21' ist geschlossen.

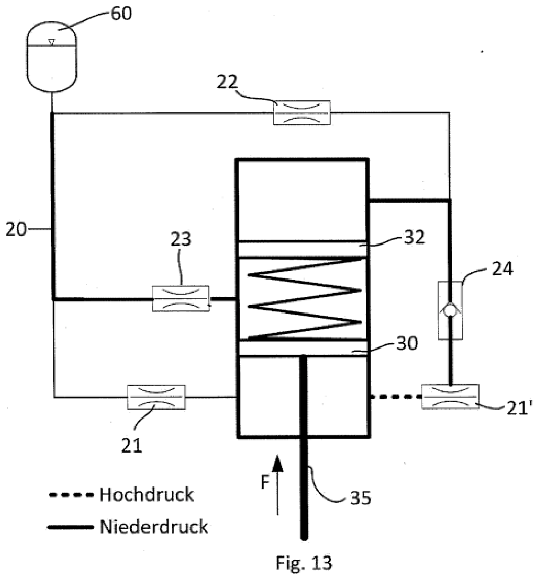

Bei einer Flexion gemäß Figur 13 und einer gewünschten Abgabe gespeicherter Energie ist das obere Ventil 22 geschlossen und das erste Zusatzventil 21' geöffnet. Das erste Ventil 21 ist geschlossen und das dritte Ventil 23 ist geöffnet, so dass sich die Feder 50 entspannen und über den Kolben 32 Fluid durch das Rückschlagventil 24 und das erste Zusatzventil 21' in die erste Fluidkammer eingeleitet werden kann.

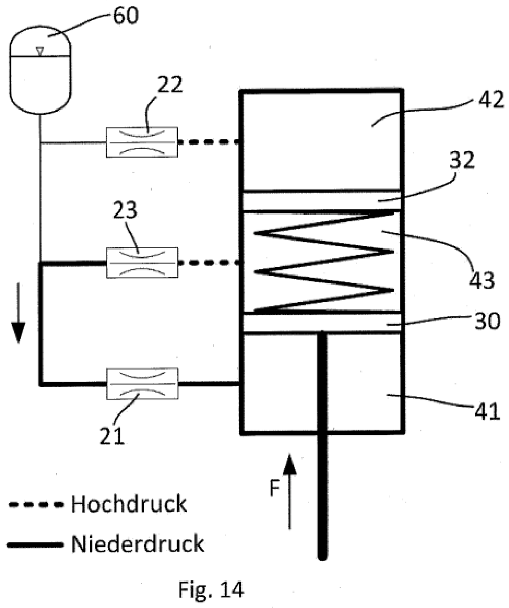

In Figur 14 ist der grundsätzliche Aufbau gemäß der Figur 1 schematisch dargestellt. Zur Speicherung von Energie bei gleichzeitiger Drosselung während einer Flexionsbewegung wird das erste Ventil 21 geöffnet, das dritte Ventil 23 zur mittleren Kammer 43 gedrosselt und das obere Ventil 22 zur oberen Fluidkammer 42 wird geschlossen. Dadurch wird einerseits die Feder 50 komprimiert, andererseits durch die Drosselung in dem Ventil 23 eine Dämpfung bei der Flexion bewirkt.

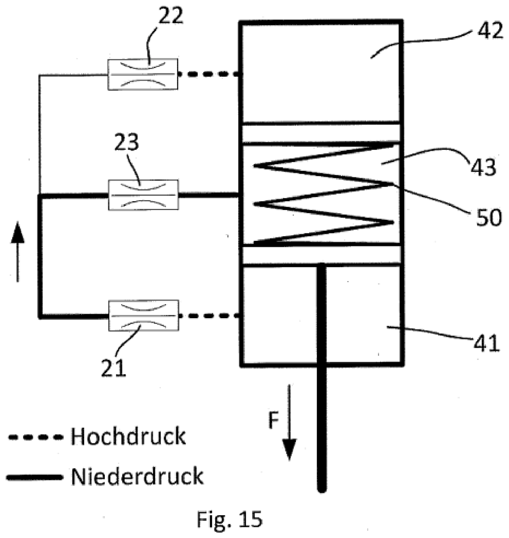

Figur 15 zeigt die Schaltung bei einer Extensionsbewegung mit Unterstützung durch den Energiespeicher 50 sowie einer Drosselung, wobei das obere Ventil 22 geschlossen ist, das mittlere Ventil 23 geöffnet ist und die Drosselung über das teilgeöffnete oder gedrosselte Ventil 21 erfolgt. Die Feder 50 kann sich entspannen, das sich verringernde Volumen der ersten Kammer 41 strömt durch die Drossel 21 in die mittlere Fluidkammer 43, so dass eine gedrosselte Extensionsbewegung erreicht wird.

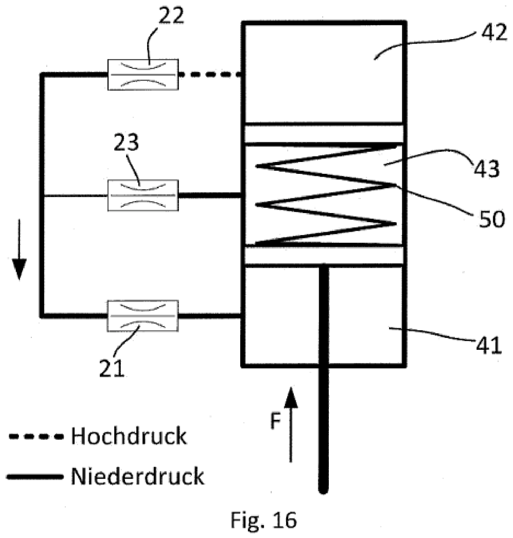

Figur 16 zeigt eine reine Dämpfung bei Flexionsbewegung mit dem geöffneten ersten Ventil 21, dem geschlossenen dritten Ventil 23 und einem gedrosselten zweiten Ventil 22. Das Hydraulikfluid strömt während der Flexion aus der oberen Kammer 42 in die untere Kammer 41, das Volumen der dritten Fluidkammer 43 verändert sich nicht, da das mittlere Ventil 23 geschlossen bleibt.

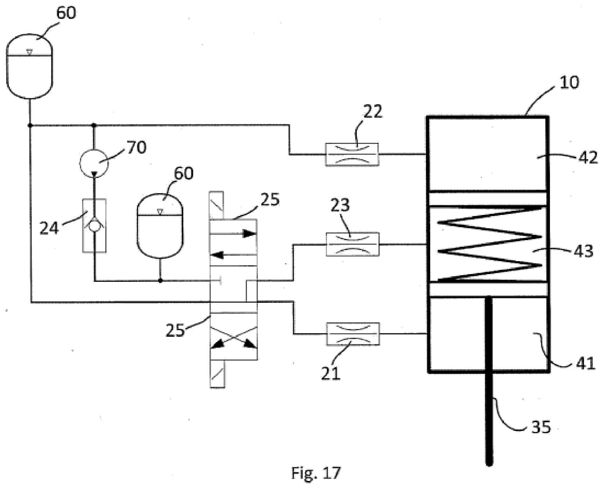

Figur 17 zeigt die Schaltanordnung gemäß Figur 2. Über das Ventil 25 kann die Pumpe 70 zur Kraftverstärkung der Extensionsbewegung verwendet werden. Die über die Kolbenstange 35 übertragene Kraft ist eine Überlagerung der Federkraft und der durch den Pumpendruck erzeugten Kraft. Dabei ist das obere Ventil 22 geschlossen, die beiden anderen Ventil 21, 23 sind offen, das Ventil 25 befindet sich in der oberen, nicht ausgekreuzten Stellung. In Flexionsrichtung können mit Hilfe der Pumpe 70 die beiden virtuell verbundenen Kolben 30, 32 und die Kolbenstange 35 verschoben werden. Dazu ist das Ventil 23 geschlossen, das obere Ventil 22 und das untere Ventil 21 sind offen, das Ventil 25 befindet sich in der unteren ausgekreuzten Stellung. Damit sich nur der untere Kolben 30 verschiebt und gleichzeitig Energie in dem elastischen Element 50 gespeichert wird, sind das mittlere Ventil 23 und das untere Ventil 21 offen und das obere Ventil 22 ist gesperrt, das Ventil 25 befindet sich in der unteren, gekreuzten Stellung.

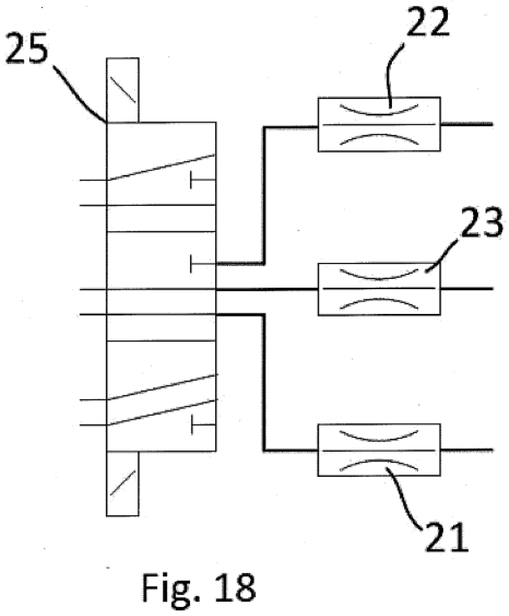

Um das Speichern sowie Abgeben von Energie während der Flexion und Extension sowie das Dämpfen in Flexions- und Extensionsrichtung zu ermöglichen, ist eine Ausgestaltung des Drei-Wege-Ventils 25 gemäß Figur 18 vorgesehen, bei der in jeder Schaltstellung des Drei-Wege-Ventils alle drei Ventile 21, 22, 23 geschaltet werden. In der mittleren Stellung wird das von der Pumpe 70 kommende Fluid der dritten, mittleren Fluidkammer 43 zugeleitet, die Leitung zu dem ersten, unteren Ventil 21 wird hergestellt, eine Kopplung mit dem oberen Ventil 22 findet nicht statt. In der rechten Stellung, wenn der untere Schaltblock mit der Pumpe 70 gekoppelt wird, wird das obere Ventil 23 mit Druckfluid beaufschlagt, die untere Fluidkammer 41 wird gesperrt. In der linken Stellung, in der der obere Schaltblock mit der Pumpe 70 gekoppelt wird, wird der Zugang zu der mittleren, eingeschlossenen Fluidkammer 43 gesperrt, die beiden äußeren Kammern 41, 42 sind miteinander gekoppelt und können mit Druckfluid von der Pumpe 70 versehen werden. Um eine aktive Kraft in beiden Richtungen ohne eine Änderung innerhalb des Energiespeichers 50 bei einem kompakten Aufbau zu ermöglichen, ist Druck in den beiden äußeren Kammern 41, 42 notwendig, wozu die Änderung in der hydraulischen Schaltung gemäß Figur 18 erforderlich ist.

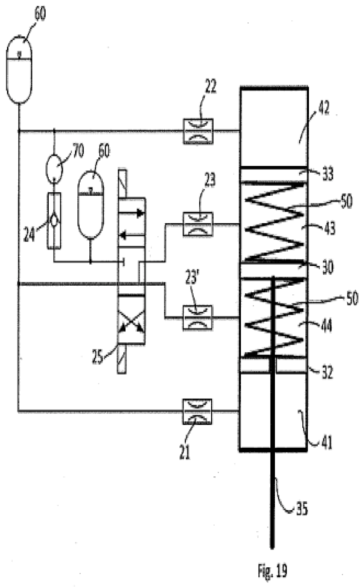

In der Figur 19 ist eine Variante der AD-Einheit gezeigt, bei der in dem Gehäuse drei Kolben 30, 32, 33 angeordnet sind, so dass insgesamt vier Fluidkammern 41, 42, 43, 44 gebildet werden. Im dargestellten Ausführungsbeispiel ist der Kolben 30, der mit der Kolbenstange 35 fest verbunden ist, zwischen den beiden weiteren Kolben 32, 33 angeordnet, wobei in den zwischen den Kolben 30, 32; 30, 33 eingeschlossenen Fluidkammern 43, 44 jeweils zumindest ein Energiespeicher 50 in Gestalt von Federn, Pneumatikkissen oder Elastomerelementen angeordnet ist. Durch das Drei-Wege-Ventil 25 lassen sich selektiv die beiden volumenvariablen, zwischen zwei Kolben ausgebildeten Kammern 43, 44 mit dem Druckfluid von der Pumpe 70 befüllen. Dazu muss das Drei-Wege-Ventil 25 entweder in die rechte oder linke Stellung bewegt werden. Ebenso lässt sich relativ leicht Druck in den beiden äußeren Kammern 41, 42 aufbauen, um ein aktive Kraft in Flexionsrichtung und Extensionsrichtung aufzubringen. Mit einer solchen Ausgestaltung der AD-Einheit ist es möglich, Energie sowohl in der Flexionsbewegung als auch in der Extensionsbewegung wahlweise zu speichern und wieder abzugeben. Durch eine Hinzuschaltung der Pumpe 70 ist es möglich, eine Kraftverstärkung bei der Abgabe von Energie in Flexions- und Extensionsrichtung bereitzustellen. Schließlich kann ein elastisches Schwingen des mittleren Kolbens 30 um die Gleichgewichtslage der Federn 43 und 44 erfolgen, das sogenannte Bouncen.

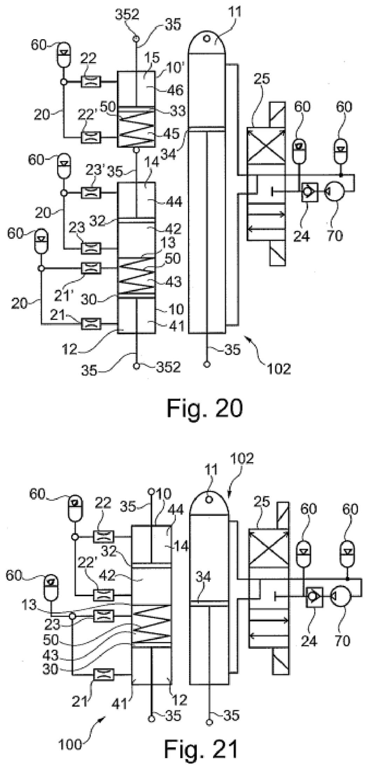

In der Figur 20 ist eine weitere Variante der Erfindung dargestellt. Die Kolben-Zylindereinheit 100 ist in drei separate Zylinderbereiche unterteilt, von denen zwei in einem Gehäuse angeordnet sind. Die untere Kolbenstange 35 ist mit dem ersten Kolben 30 verbunden. Die erste Fluidkammer 41 befindet sich zwischen der unteren Zylinderwand und dem Kolben 30. Auf der gegenüberliegenden Seite des Kolbens 30 ist ein Energiespeicher 50 in einer zweiten Fluidkammer 43 angeordnet. Der Energiespeicher 50 in Gestalt einer Feder stützt sich einerseits an dem Kolben 30 und andererseits an einer Trennwand 13 ab. Auf der anderen Seite der Trennwand 13 befindet sich ein zweiter, längsverschieblicher Kolben 32, der in einem zweiten, strömungstechnisch von dem ersten Zylinder 12 entkoppelten Zylinder 14 angeordnet ist und das Zylindervolumen in zwei Fluidkammern 42, 44 unterteilt. Innerhalb der beiden Fluidkammern 42, 44 ist kein Energiespeicher angeordnet. Der zweite Kolben 32 ist über eine Kolbenstange 35 mit einem separaten Zylindergehäuse 10' gekoppelt, das über die Kolbenstange 35 des zweiten Kolbens 32 verschieblich zu dem ersten Gehäuse 10 gelagert ist. Innerhalb des zweiten Gehäuses 10' ist ein dritter Kolben 33 verschieblich in einem dritten Zylinder 15 gelagert. Der dritte Zylinder 15 ist strömungstechnisch von den anderen Zylindern 12, 14 entkoppelt. Aus dem zweiten Gehäuse 10' ragt eine dritte Kolbenstange 35, die an dem dritten Kolben 33 festgelegt ist, heraus und ist an einer Komponente der orthetischen oder prothetischen Vorrichtung festlegbar. Der Kolben 33 unterteilt den dritten Zylinder 15 in zwei Fluidkammern 45, 46, in der dem ersten Gehäuse 10 zugewandten Fluidkammer 45, die der dritten Kolbenstange 35 abgewandt liegt, ist ein zweiter Energiespeicher 50 angeordnet. Die drei Kolben 30, 32, 33 sind somit in Reihe geschaltet, zwei in einem gemeinsamen Gehäuse 10 mit einer Trennwand 13, der dritte in einem relativ zu dem ersten Gehäuse 10 beweglich an einem Kolben gelagerten zweiten Gehäuse 10'. Beide Gehäuse 10, 10' können zu einer Baueinheit zusammengefasst werden. Die Befestigung der Kolben-Zylindereinheit 100 erfolgt über die beiden aus den Gehäusen 10, 10' endseitig herausragenden Kolbenstangen 35 an den Befestigungselementen 352.

Jede Fluidkammer 41, 42, 43, 44, 45, 46 weist eine mit einem Ventil 21, 21', 22, 22', 23, 23' versehene Zugangsöffnung auf. Die beiden jeweils durch den beweglichen Kolben 30, 32, 33 getrennten Fluidkammern 41, 43; 42, 44; 45, 46 sind über eine Fluidleitung 20 und einem zugeordneten Ausgleichsvolumen 60, das druckbeaufschlagbar sein kann, verbunden. Über die Ventile 21, 21', 22, 22', 23, 23' kann das Dämpfungsverhalten sowie das Speicherverhalten der Energiespeicher 50 beeinflusst werden.

Parallel zu der ersten Kolben-Zylinder-Einheit 100 ist eine zweite Kolben-Zylindereinheit 102 angeordnet, die als reiner Aktuator ausgebildet ist. Die Kräfte der beiden Kolben-Zylinder-Einheiten 100, 102 überlagern sich. Ein Kolben 34 ist in einem Gehäuse verschieblich gelagert und trennt zwei Fluidkammern voneinander. Eine Pumpe 70 ist über ein Rückschlagventil 24 und zwei Ausgleichsvolumina 60 mit der zweiten Kolben-Zylindereinheit gekoppelt. Ein Drei-Wege-Ventil 25 ist zwischen der Pumpe 70 und den Fluidkammern in der zweiten Kolben-Zylinder-Einheit 102 angeordnet und ermöglicht eine Druckbeaufschlagung entweder einer Extensionskammer oder einer Flexionskammer, so dass eine aktive Krafteinleitung je nach Stellung des Drei-Wege-Ventils 25 erfolgt. In der dargestellten mittleren Stellung findet kein Antrieb statt, wird das Drei-Wege-Ventil 25 nach unten verfahren, findet eine Extension statt, wird das Drei-Wege-Ventil 25 nach oben verfahren, findet eine Flexion statt, bei der die Kolbenstange 35 in das Gehäuse der zweiten Kolben-Zylinder-Einheit 102 hineingefahren wird.

Eine Variante der Erfindung gemäß Figur 20 ist in der Figur 21 dargestellt, bei der ebenfalls eine Trennung eines aktiven Aktuators über die zweite Kolben-Zylinder-Einheit 102 und einer ersten Kolben-Zylinder-Einheit 100 vorgenommen wurde. Im Unterschied zu der Ausführungsform gemäß Figur 20 ist eine Energiespeicherung nur in Flexionsrichtung und eine Energieabgabe aus dem Speicher nur in Extensionsrichtung vorgesehen. Das Gehäuse 10 der ersten Kolben-Zylinder-Einheit 100 ist durch eine Trennwand 13 unterteilt, es sind zwei strömungstechnisch getrennte Zylinder 12, 14 ausgebildet. An beiden Enden des Gehäuses ragen Kolbenstangen 35 aus dem Gehäuse 10 heraus. Die Kolbenstange 35 ist mit einem ersten Kolben 30 verbunden, der eine erste Fluidkammer 41 und eine zweite Fluidkammer 43 voneinander trennt. In der zweiten Fluidkammer 43 ist ein mechanischer Energiespeicher 50 in Gestalt einer Feder angeordnet. Jenseits der Trennwand 13 ist der zweite Kolben 32 längsverschieblich angeordnet, der ebenfalls zwei Fluidkammern 42, 44 voneinander trennt. Über Ventile 21, 23, 22', 22 ist der Fluidstrom zwischen den von den Kolben 30, 32 getrennten Fluidkammern 41, 43; 42, 44 regelbar. Jeder Fluidkammerpaarung ist ein Ausgleichsvolumen 60 zugeordnet. Mit der dargestellten Ausführungsform wird eine Trennung von Einspeicherung mechanischer Energie und Dämpfung sowie eine separate Aktuierung über eine Pumpe 70 vorgenommen.

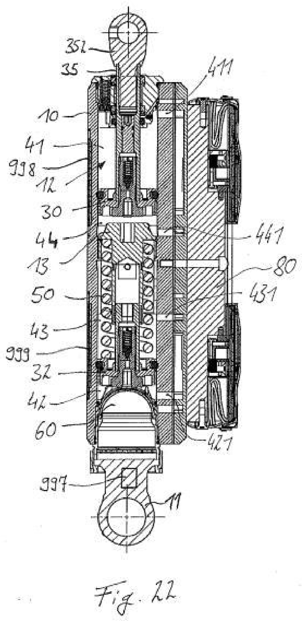

Figur 22 zeigt eine Schnittdarstellung durch eine AD-Einheit mit vier Fluidkammern 41, 42, 43, 44. Über eine Kolbenstange 35 ist ein erster Kolben 30 in einem Zylinder 12 geführt, der durch das Gehäuse 10 ausgebildet wird. Das dem ersten Kolben 30 abgewandte Ende 352 der Kolbenstange 35 ragt aus dem Gehäuse 10 heraus und dient zur Befestigung an einer orthetischen oder prothetischen Vorrichtung. Der erste Kolben 30 trennt zwei Fluidkammern 41, 44 voneinander, wobei der Abschluss der Fluidkammer 44 durch eine fixierte Trennwand 13 innerhalb des Zylinders 12 ausgebildet wird. Auf der dem ersten Kolben 30 abgewandten Seite der Wand 13 ist ein weiterer Zylinderabschnitt ausgebildet, in dem ein zweiter Kolben 32 verschieblich gelagert ist. Der zweite Kolben 32 bildet ebenfalls zwei Fluidkammern 42, 43 aus, die zwischen der Trennwand 13 und dem zweiten Kolben 32 befindliche Fluidkammer 43 ist mit einem Energiespeicher 50 in Gestalt einer Spiralfeder versehen. Alle Fluidkammern 41, 42, 43, 44 sind mit Zugangsöffnungen 411, 421, 431, 441 versehen, um über eine entsprechende Steuereinheit 80 mit nicht dargestellten Ventilen die unterschiedlichen hydraulischen Verschaltungen und Änderungen in dem Dämpferverhalten, dem Speicherverhalten sowie die Aktuierungsverhalten zu bewirken. In die Einheit ist ein Ausgleichvolumen 60 integriert, die Anbindung 11 dient der Befestigung an einer prothetischen oder orthetischen Einrichtung. In dieses Element kann ein Kraftsensor 997 integriert werden, der Signale für die Steuerung 80 liefert. Weiterhin verfügt die Einheit über integrierte Sensoren 998, 999 zur Messung der Kolbenpositionen. Der Sensor 998 misst die Position des Arbeitskolbens und gibt somit Aufschluss über den Gelenkswinkel, der Sensor 999 misst die Position des Speicherkolbens und gibt Aufschluss über die gespeicherte Energie;

Die erfindungsgemäße AD-Einheit kann sowohl als reiner Dämpfer, als auch als reiner Aktuator, als auch als Kombination aus Dämpfer mit Aktuator eingesetzt werden. Bei einer Ausgestaltung als reiner Aktuator muss keine Dämpfung vorhanden sein, bei einer Ausgestaltung als reiner Dämpfer muss keine Aktuierung oder Energiespeicherung vorhanden sein. Grundsätzlich besteht die Möglichkeit, die AD-Einheit so auszugestalten, dass zumindest eine der drei Optionen der Verwendung verwirklicht wird.

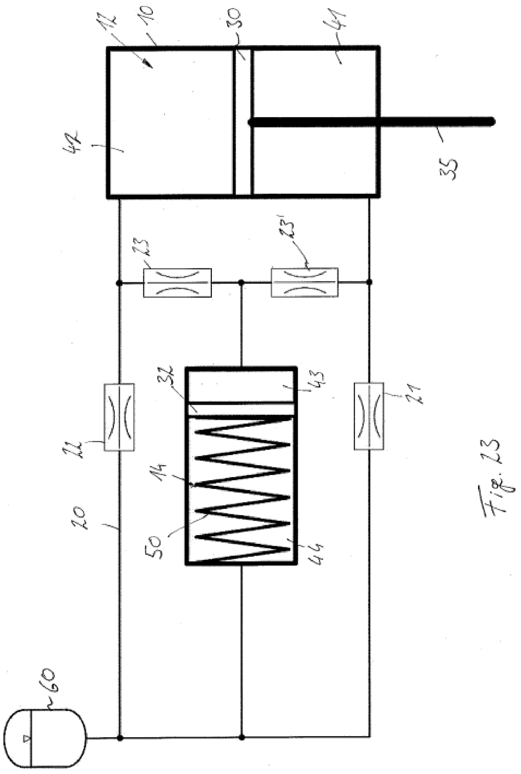

Eine weitere Variante der Erfindung ist in der Figur 23 gezeigt, in der eine schematische Darstellung einer AD-Einheit 100 mit einem längsbeweglichen Kolben 30 in einem Zylinder 12 dargestellt ist. Eine AD-Einheit gemäß Figur 23 ist in der Lage, Energie in beiden Bewegungsrichtungen zu speichern und beliebig wieder abzugeben. Der Kolben 30 ist über die Kolbenstange 35 mechanisch mit einer nicht dargestellten orthopädischen oder orthetischen Einrichtung gekoppelt. Zwei Fluidkammern 41, 42 werden durch den Kolben 30 voneinander getrennt. Beide Fluidkammern 41, 42 sind über Fluidleitungen 20, in denen Schalt- oder Stellventile 21 angeordnet sind, miteinander verbunden. Ein Ausgleichsvolumen 60 ist an der Fluidleitung 20 angeschlossen, um Volumenschwankungen, insbesondere durch das Einfahren und Ausfahren der Kolbenstange 35 und gegebenenfalls Temperaturschwankungen oder Leckagen, ausgleichen zu können. Über die Stellventile 21, 22 kann der Volumenstrom zwischen den Fluidkammern 41, 42 gesteuert werden, die Ventile 21, 22 werden über eine nicht dargestellte Steuerungseinrichtung 80, die bevorzugt computergesteuert ist, verstellt.

Darüber hinaus ist ein zweiter Zylinder 14 strömungstechnisch mit den Fluidkammern 41, 42 gekoppelt, in dem zweiten Zylinder 14 ist ein weiterer Kolben 32 verschieblich gelagert und wird über ein Energiespeicher 50 in Gestalt eines Federelementes oder Elastomerelementes gegen einen Fluiddruck vorbelastet. Der zweite Kolben 32 unterteilt den zweiten Zylinder 14 in zwei weitere Fluidkammern 43, 44, die jeweils mit einer Fluidleitung verbunden sind. Stellventile 23, 23' sind in einer Verbindungsleitung zwischen den Fluidleitungen 20 von den ersten Fluidkammern 41, 42 angeordnet und steuern den Zustrom und den Abstrom des Fluides, insbesondere des Hydraulikfluides, in und aus der druckbeaufschlagten Fluidkammer 43. Soll der Energiespeicher 50 aufgeladen werden, wird durch eine entsprechende Ventilstellung dafür gesorgt, dass aufgrund einer Bewegung des ersten Kolbens 30 das Fluid in die volumenveränderbare Fluidkammer 43 geleitet wird. Wird beispielsweise die Kolbenstange 35 eingefahren, bewegt sich der Kolben 30 nach oben. Das untere erste Stellventil 21 ist geöffnet, um ein Fluid aus der Kammer 44 in die untere Fluidkammer 41 einströmen zu lassen. Das untere Stellventil 23' in der Verbindungsleitung ist geschlossen, das obere Stellventil 23 in der Verbindungsleitung ist geöffnet und das Stellventil 22 in der Fluidleitung 20 ist geschlossen, sodass das Fluid aus der oberen Fluidkammer 42 über die Fluidleitung 20 durch das obere Stellventil 23 in der Verbindungsleitung in die volumenveränderbare Fluidkammer 43 hineingepresst wird, wodurch die Feder 50 komprimiert und dadurch Energie gespeichert wird. Um das Energieniveau beibehalten zu können, bleiben die Stellventile 23, 23' in der Verbindungsleitung geschlossen, soll die Aktuator-Dämpfereinheit 100 zum Antreiben genutzt werden, wird das jeweilige Stellventil 23, 23' geöffnet, um eine entsprechende Aktuierung des ersten Kolbens 30 und dann mit der Kolbenstange 35 zu bewirken.

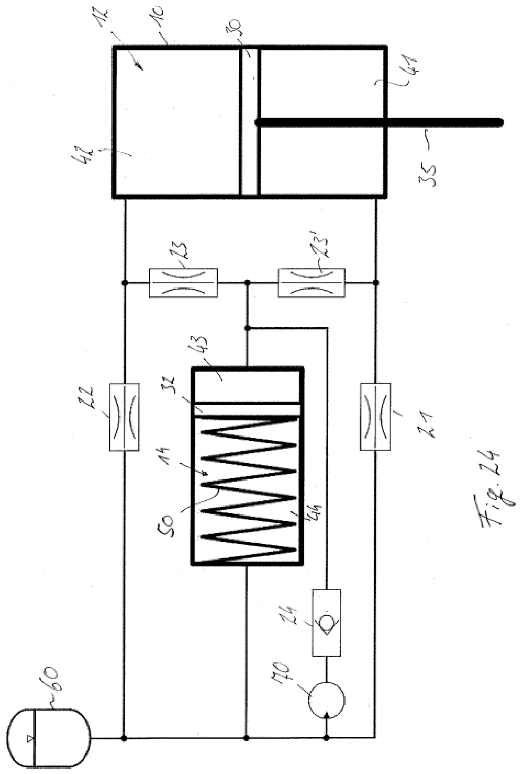

Eine Variante der Erfindung gemäß Figur 23 ist in der Figur 24 gezeigt, bei der zusätzlich zu der Ausgestaltung gemäß Figur 23 eine Pumpe 70 mit einem Rückschlagventil 24 mit dem zweiten Zylinder 14 strömungstechnisch gekoppelt ist. Über die Pumpe 70 ist es möglich, unabhängig von dem ersten Kolben 30 und einer Bewegung des Kolbens 30 das Energieniveau innerhalb des Energiespeichers 50 aufzuladen, um eine ausreichend große Energiemenge bereitstellen zu können, wenn dies erforderlich ist. Die Pumpe 70 kann beispielsweise über einen Elektromotor angetrieben werden und mit einem Computer oder einer elektronischen Steuerungseinrichtung gekoppelt sein, um zu steuern, ob und wie viel Energie gespeichert oder abgegeben werden soll. Man kann zusätzlich den Aktuator aktiv bewegen, um eine Flexion und Extension zu bewirken, wobei der Energiespeicher 50 als seriell elastisches Element wirkt.

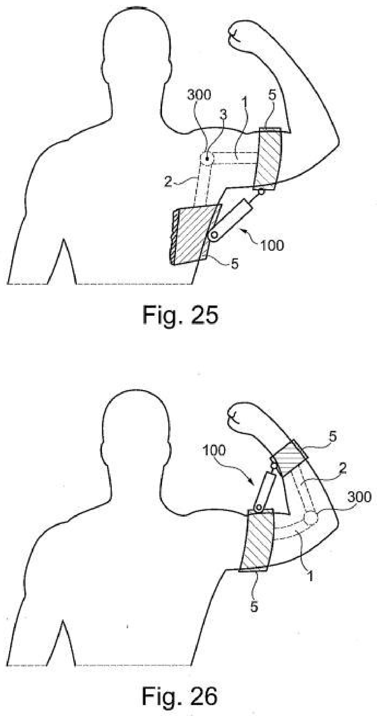

Figur 25 zeigt ein erstes Anwendungsbeispiel einer Aktuator-Dämpfereinheit 100 zur Unterstützung eines Schultergelenks. Dabei verbindet die Aktuator-Dämpfereinheit 100 ein optionales Oberteil 1 über eine Befestigungseinrichtung 5 in Gestalt einer Oberarmmanschette mit einem optionalen Unterteil 2, das ebenfalls über eine Befestigungseinrichtung 5 in Gestalt einer Thoraxschale 5 an dem Thorax eines Patienten festgelegt ist. Das Unterteil 2 ist mit dem Oberteil 1 über eine Gelenkeinrichtung 300 schwenkbar um eine Schwenkachse 3 verbunden. Das Oberteil 1 und das Unterteil 2 sind als Schiene einer Orthese ausgebildet. Beide Befestigungseinrichtungen 5 sind mit Aufnahmeeinrichtungen für die Kolbenstange 35 und das Gehäuse 10 versehen, sodass beim Einfahren oder Ausfahren der Kolbenstange 35 in das Gehäuse 10 der Arm von dem Thorax abgespreizt oder herangezogen wird. Alternativ zu einer Kopplung der Befestigungseinrichtungen 5 über einen Gelenkmechanismus 300 mit der Schwenkachse 3 und dem Oberteil 1 und dem Unterteil 2 besteht die Möglichkeit, dass der Kraftrückschluss direkt über den Körper des Patienten erfolgt, also über die Skelettstruktur, sodass das Oberteil und das Unterteil durch die Befestigungseinrichtungen 5 verwirklicht werden. Die Kraftübertragung von der Thoraxschale 5 zu dem Boden erfolgt entweder über den Körper des Anwenders oder über eine den Oberkörper und die untere Extremität umfassende Orthesenkonstruktion, ähnlich der eines Exoskelettes.

Figur 26 zeigt eine Variante in der Anwendung, bei der die Aktuator-Dämpfereinheit 100 zur Unterstützung des Ellenbogengelenkes eingesetzt wird. Dabei verwendet die Aktuator-Dämpfereinrichtung 100 eine erste Befestigungseinrichtung 5 in Gestalt einer Unterarmschale mit einer zweiten Befestigungseinrichtung 5 in Gestalt einer Oberarmschale oder einer Oberarmmanschette. Auch hier kann die orthetische oder prothetische Vorrichtung allein aus dem Aktuator-Dämpfereinrichtung 100 und den beiden Befestigungseinrichtungen 5 bestehen, optional kann der Kraftrückschluss über ein Oberteil 1 und ein Unterteil 2, die über eine Gelenkeinrichtung 300 miteinander verbunden sind, erfolgen. Das Oberteil 1 und das Unterteil 2 können jeweils als eine Schiene ausgebildet sein. Es ist auch möglich, die Ausführungsform gemäß Figur 26 mit der Ausführungsform gemäß Figur 25 zu kombinieren, sodass neben einem unterstützten Schultergelenk gemäß Figur 25 auch ein unterstütztes Ellenbogengelenk erzielt werden kann. Die weitere Krafteinleitung in den Boden erfolgt vorteilhafterweise über eine weitere, nicht dargestellte Orthesenkonstruktion oder ein Exoskelett.

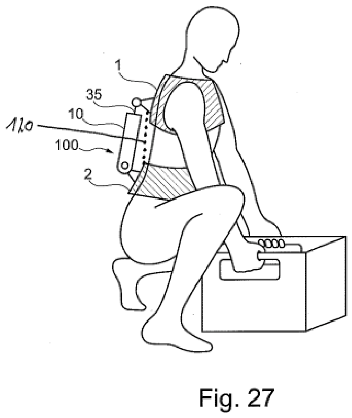

Figur 27 zeigt eine weitere Variante der Erfindung, bei der die Aktuator-Dämpfereinheit 100 zur Unterstützung des Rumpfes eingesetzt wird. Das Gehäuse 10 ist über eine erste Lagerstelle mit einem Oberteil 1 in Gestalt einer proximalen Körperschale und über eine zweite Lagerstelle mit einem Unterteil 2 in Gestalt einer distalen Körperschale im Bereich der Lendenwirbelsäule verbunden. Im dargestellten Ausführungsbeispiel ist die Kolbenstange 35 mit dem Oberteil 1 verbunden, grundsätzlich ist jedoch auch eine umgekehrte Zuordnung vorgesehen und möglich, sodass die Kolbenstange 35 auch an dem Unterteil 2 angeordnet sein kann. Weiterhin ist eine Kaskadierung mehrerer Aktuator-Dämpfereinheiten 100 über mehrere Segmente entlang des Rückens bzw. der Rückenwirbelsäule möglich, ggf. kann die Aktuator-Dämpfereinheit 100 auch über zwei Kolbenstangen über mehrere Gelenke wirkend ausgebildet sein. Eine mechanische Kopplung 120 zwischen dem Oberteil 1 und dem Unterteil 2 zur Realisierung eines Widerlagers für die Aktuatorkraft ist möglich und in der Figur durch eine strichlierte Linie dargestellt.

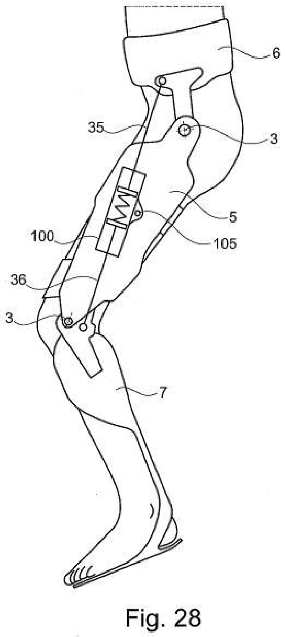

Figur 28 zeigt eine orthetische Einrichtung in Gestalt einer HKAFO (hip-knee-ankle-foot-orthoses), bei der die Aktuator-Dämpfereinheit 100 an einer Oberschenkelschale 5 befestigt ist. Die Aktuator-Dämpfereinheit 100 weist zwei separate Kolben 30, 32 auf die über eine Feder oder ein Elastomerelement miteinander gekoppelt und jeweils mit einer Kolbenstange 35, 36 verbunden sind. Die Kolbenstangen 35, 36 greifen einmal an einer Hüftschale 6 und an einer Unterschenkelschale 7 an. Die Hüftschale 6 ist über ein Gelenk um eine Gelenkachse 3 schwenkbar an der Oberschenkelschale 5 gelagert. Die Drehachse 3 liegt auf der Höhe der natürlichen Hüftgelenkachse, eine Verschwenkbewegung erfolgt durch Einfahren oder Ausfahren der ersten Kolbenstange 35 in das oder aus dem Gehäuse 10. Die zweite Kolbenstange 36 ist an der Unterschenkelschiene 7 mit einem optionalen, angeformten Fußteil gelagert. Die Unterschenkelschiene 7 ist über ein Gelenk um eine Schwenkachse 3 schwenkbar relativ zu der Oberschenkelschale 5 gelagert. Auch diese Schwenkachse 3 liegt auf der Höhe der natürlichen Gelenkachse die orthetische Einrichtung übergreift mehrere Gelenke, vorliegend das Kniegelenk und das Hüftgelenk, wobei die Aktuator-Dämpfereinheit 100 über einen Anbindungspunkt 105 drehbar an der Oberschenkelschale 5 gelagert ist. Je nach Betätigung des Aktuators werden die unterschiedlichen Einfahr- und Ausfahrbewegungen der Kolbenstangen 35, 36 bewirkt, was zu einer Verschwenkung der jeweiligen Komponenten 5, 6, 7 zueinander führt. Ebenso kann durch entsprechende Schaltung von Ventilen oder Aktivierung von Magnetfeldern beim Einsatz magnetorheologischer Fluide eine Bewegungsdämpfung erreicht werden. Das Prinzip einer Orthese oder Prothese mit einem Aktuator, der mehrere Gelenke übergreift, lässt sich auch für andere Körpersegmente anwenden, insbesondere für das Knie- und Knöchelgelenk, für Rumpfsegmente oder auch für orthetische oder prothetische Einrichtungen an der oberen Extremität.

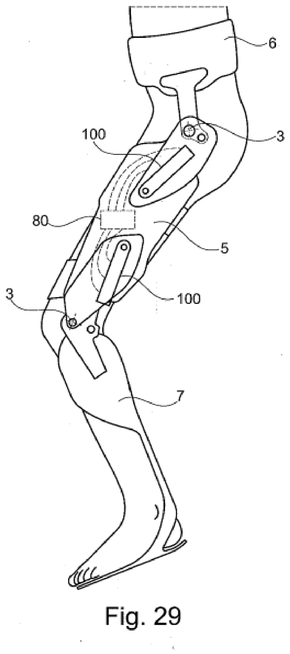

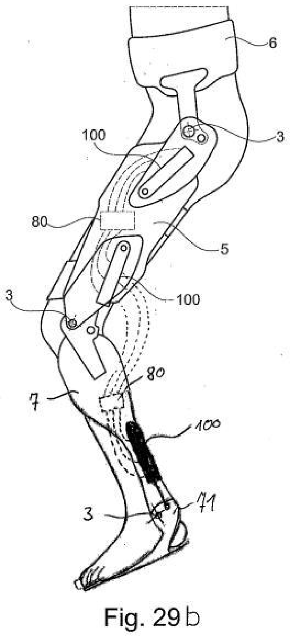

Eine Variante der Ausgestaltung einer gelenkübergreifenden orthetischen Einrichtung ist in der Figur 29 dargestellt, bei der statt eines einzelnen Aktuators bzw. einer einzelnen Aktuator-Dämpfereinheit 100 zwei Aktuator-Dämpfereinheiten 100 an einer Oberschenkelschale 5 festgelegt sind. Die einzelnen Aktuator-Dämpfereinheiten 100 sind ggf. hydraulisch miteinander gekoppelt, wobei bei der dargestellten hüftübergreifenden Beinorthese ein erster Aktuator 100 für die Bewegung der Oberschenkelschale 5 relativ zu der Hüftschale 6 oder Manschette 6 im Bereich des natürlichen Hüftgelenkes eingesetzt wird, während der zweite Aktuator 100 die Kniebewegung durch eine Verlagerung der Unterschenkelschiene 7 relativ zu der Oberschenkelschale 5 um das Drehgelenk 3 bewirkt. Beide Aktuatoren 100 können über optionale hydraulische Leitungen, die gestrichelt dargestellt sind, miteinander hydraulisch verschaltet sein. Dazu kann eine Steuerungseinrichtung 80 oder ein Ventilsteuerblock in den Leitungen vorhanden sein, wobei durch den Ventilsteuerblock 80 verschiedene Verschaltungsvarianten einen Energietransfer zwischen den jeweiligen Aktuatoren 100 ermöglichen. Eine derartige Kopplung ist auch zwischen weiteren Aktuatoren an anderen Stellen, bzw. am Knie oder am Fuß oder zwischen beiden Beinen möglich. Die Steuerung 80 kann computergestützt arbeiten und Sensorsignale verarbeiten.

Figur 29b zeigt eine Variante der Figur 29 mit einem beweglich um eine Schwenkachse 3 an der Unterschenkelschiene 7 gelagerten Fußteil 71, das über eine AD-Einheit 100 in Richtung einer Plantarflexion und einer Dorsalflexion bewegt werden kann. Die an der Unterschenkelschale 7 gelagerte AD-Einheit 100 ist über eine separate, ggf. mit der an Oberschenkelschale 5 gelagerten Steuerung 80 gekoppelten Steuerung 80 gesteuert. Die Hydraulikströme können zwischen den AD-Einheiten 100 gekoppelt sein, um eine Kraftübertragung über die einzelne AD-Einheit 100 hinaus zu ermöglichen, so dass ggf. weniger Pumpen oder nur ein Pumpe eingesetzt werden können. Mit der Variante der Figur 29b wird das Fußteil aktuiert oder in seiner Verschwenkung relativ zu der Unterschenkelschale 7 gedämpft oder blockirrt.

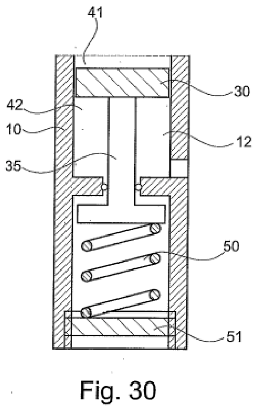

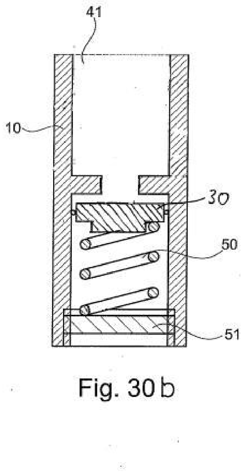

Figur 30 zeigt eine alternative Form des Energiespeichers 50, bei dem die Feder 50 des Federspeichers außerhalb des Fluidstroms, insbesondere außerhalb eines Hydraulikfluidstroms liegt. Dazu ist die Kolbenstange 35 gegenüber dem Energiespeicher 50 in Gestalt der Feder über einen Dichtring abgedichtet, so dass die Feder 50 nicht in Kontakt mit dem Hydraulikfluid, insbesondere Hydrauliköl gelangt. Dies hat den Vorteil, dass der Energiespeicher 50 auch aus einem Material bestehen kann, das mit einem Hydraulikfluid unverträglich ist. Darüber hinaus kann der Energiespeicher 50 einfach gewechselt werden, ohne dass ein Ölkreislauf geöffnet werden muss. Dazu wird lediglich eine Einstell- und Rückhaltescheibe 51 aus dem Gehäuse 10 entfernt, so dass die Feder 50 oder der Energiespeicher 50 einfach entnommen werden kann, Über die Scheibe 51 ist es auch möglich, die Vorspannung des Energiespeichers 50 zu verändern, beispielsweise wenn diese Scheibe 51 in das Gehäuse 10 eingeschraubt ist. Ein entsprechendes Gewinde ist in der Figur 30 angedeutet. Der Öldruck der jeweiligen Fluidkammer 42, 41 wirkt sich auf den Kolben 30 aus, der über den Stempel am Ende der Kolbenstange 35 den Energiespeicher 50 in Gestalt der Feder komprimiert. Das Öl aus der zweiten Fluidkammer 42 gelangt aus der Niederdruckkammer über eine Öffnung in den Niederdruckkreislauf, in dem sich auch ein Ausgleichsvolumen befindet. Das Ausgleichsvolumen nimmt die durch die Kolbenstange 35 verdrängte Ölmenge auf. Durch den Einsatz unterschiedlich steifer Federn oder Energiespeicher 50 kann die Speichercharakteristik beeinflusst und an die individuellen Bedürfnisse unterschiedlicher Patienten angepasst werden. Eine weitere Anpassung an die individuellen Wünsche der Patienten kann über die Einstellschraube oder Einstellscheibe 51 erfolgen.

Eine Variante der Figur 30 ist in der Figur 30b gezeigt, bei der der Kolben 30 entgegen einer Federkraft durch den Energiespeicher 50 in dem Gehäuse 10 gelagert ist. Der Kolben 30 ist dabei gegenüber der Gehäuseinnenwand abgedichtet, so dass das Fluid aus der Kammer 41 direkt über den Kolben die Feder 50 komprimiert und keine Kolbenstange 35, kein Stempel und kein Auslasskanal für aus der unteren Kammer 42 verdrängtes Fluid benötigt wird.

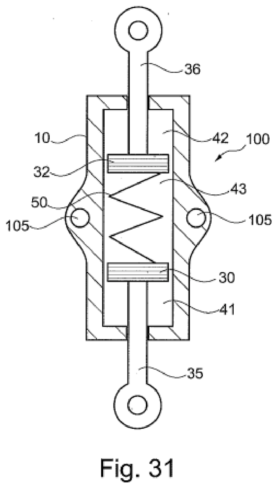

Figur 31 zeigt eine Einzeldarstellung einer Aktuator-Dämpfereinheit mit zwei Kolben 30, 32, die insgesamt drei Fluidkammern 41, 42, 43 ausbilden. Zwischen den beiden Kolben 30, 32 ist eine Feder als Energiespeicher 50 angeordnet. Eine hydraulische Verschaltung ist nicht dargestellt. In der dargestellten Ausführungsform sind zwei Kolbenstangen 35, 36 vorhanden, die über Anbindungspunkte an ihren äußeren Enden an Prothesen- oder Orthesenstrukturteilen festgelegt werden können. Das Gehäuse 10 der Aktuator-Dämpfereinheit 100 ist über Anbindungspunkte 105 an eine Orthesenstruktur oder Prothesenstruktur befestigbar, wodurch ein Aktuator 100, der mehrere Gelenke übergreift, gemäß der Figur 28 realisiert werden kann. Wird nur das Gehäuse 10 und eine der Kolbenstangen 35, 36 an Orthesenstrukturteilen oder Prothesenstrukturteilen festgelegt, so wirkt die Aktuator-Dämpfereinheit 100 nur auf diese Verbindung zwischen den beiden Strukturteilen, die zweite Kolbenstange ist dann mit keinem Strukturbauteil verbunden und dient nur dem Volumenausgleich.

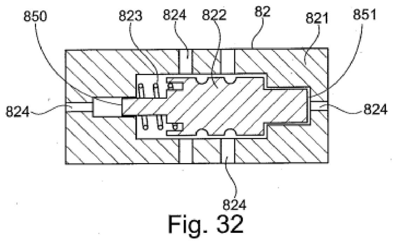

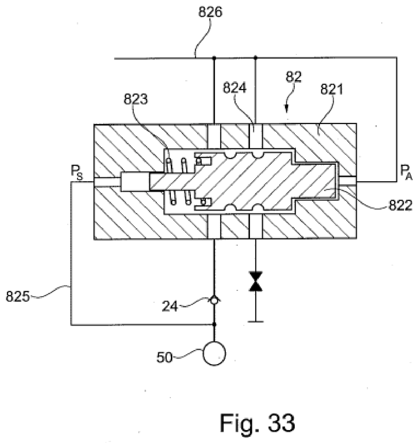

Figur 32 zeigt eine schematische Schnittdarstellung eines mechanischen Druckregelventils 82 für einen Druckspeicher mit einem Ventilkörper 821 und einem beweglich in dem Ventilkörper 821 gelagerten Schaltelement 822, das über eine Feder 823 in einer Ausgangsstellung gelagert ist. In dem Ventilkörper 821 sind mehrere Anschlussbohrungen 824 für ein Druckfluid, insbesondere ein Hydraulikfluid, angeordnet, die mit Fluidleitungen verbunden sind. Durch unterschiedliche Gestaltung der Querschnitte 850, 851 lässt sich eine Hysterese in der Schaltfunktion realisieren bzw. der Effekt der Kraft der Rückstellfeder teilweise kompensieren. Die hydraulische Verschaltung des Druckregelventils 82 ist in der Figur 33 gezeigt. Das bewegliche Schaltelement 822 ist über eine Druckleitung 825 mit einem Fluiddruck pₛ aus dem Druckspeicher 50 beaufschlagt. Der Fluiddruck pₛ unterstützt die Druckfeder 823 und drückt das Schaltelement 822 in die dargestellte Ausgangsstellung. Wird über eine Arbeitsdruckleitung 826 Hydraulikfluid aus der Aktuator-Dämpfereinheit, die nicht dargestellt ist, dem Druckregelventil 82 zugeführt, so liegt ein Arbeitsdruck p_{A} an dem Schaltelement 822 an. Der Arbeitsdruck p_{A} wirkt gegen den Speicherdruck p_{S} und gegen die Federkraft der Feder 823. Innerhalb des beweglichen Schaltelementes 822 sind umlaufende Nuten angeordnet, die in der Ausgangsstellung einen Durchlass von der Arbeitsdruckseite zu einer Niederdruckseite ermöglichen. Ist der Arbeitsdruck p_{A} größer als der Speicherdruck p_{S}, wird das Schaltelement 822 nach links geschoben, so dass die linke Nut innerhalb des Schaltelementes 822 zu den linken Bohrungen innerhalb des Ventilkörpers 862 ausgerichtet wird. Dadurch ergibt sich eine Strömungsverbindung von der Arbeitsdruckseite zu dem Druckspeicher 50, so dass das Arbeitsfluid nicht auf die Niederdruckseite p_{L} gelangt, sondern den Druckspeicher 50 auflädt. Ein Zurückströmen direkt durch die strömungstechnische Verbindung wird durch Rückschlagventil 24 verhindert. Wird der Speicherdruck p_{S} zu groß, wird das Schaltelement 822 wieder nach rechts verschoben, so dass ohne Abzweigung eines Druckes für den Druckspeicher 50 das Fluid durch das Druckregelventil 82 hindurchströmen kann. Ein solches Druckregelventil 82 benötigt keinen elektronischen Steuerungsaufwand und kann ohne externe Energiezufuhr betrieben werden.

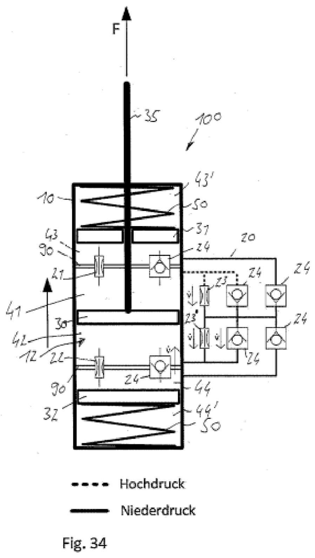

In den Figuren 34 bis 39 ist eine weitere Variante der Erfindung schematisch dargestellt. Die Aktuator-Dämpfereinheit 100 weist ein Gehäuse 10 mit einem darin ausgebildeten Zylinder 12 auf, in dem ein erster Kolben 30 als Arbeitskolben verschieblich gelagert ist. Der Kolben 30 ist über eine Kolbenstange 35, die aus dem Gehäuse 10 hinausführt, mit einer nicht dargestellten prothetischen oder orthetischen Einrichtung gekoppelt oder zumindest koppelbar, ebenso ist das Gehäuse an einem anderen Teil der orthetischen oder prothetischen Einrichtung festlegbar. Innerhalb des Zylinders 12 sind zwei Sperrwände 90 angeordnet, die den Zylinder 12 in insgesamt drei Kammern unterteilt, nämlich in eine mittlere Hauptkammer und zwei äußere Nebenkammern. Innerhalb dieser Kammern sind jeweils ein bewegliche gelagerter Kolben 30, 31, 32 gelagert. Der mit der Kolbenstange 35 verbundene Kolben 30 ist in der mittleren Kammer angeordnet und unterteilt diese Hauptkammer in zwei Fluidkammern 41, 42. In den von den Sperrwänden 90 abgetrennten Nebenkammern sind federbelastete Kolben 31, 32 angeordnet, die im dargestellten Ausführungsbeispiel auf der der Hauptkammer zugewandten Seite der jeweiligen Sperrwand 90 angeordnet sind. Die Federn 50, die sich sowohl an dem jeweiligen Kolben 31, 32 als auch der Zylinderaußenwand abstützen, sind auf der dem mit der Kolbenstange 35 verbundenen mittleren Arbeitskolben 30 abgewandten Seite angeordnet.

Innerhalb der Sperrwände 90 sind jeweils ein Stellventil 21, 22 und ein Rückschlagventil 24 angeordnet.

Die Federkolben 31, 32 unterteilen die jeweilige Nebenkammer in eine mit dem Hydraulikfluid befüllbare Fluidkammer 43, 44 und eine fluidfreie Kammer 43', 44'. Innerhalb der fluidfreien Kammern 43', 44' ist der Energiespeicher 50, im dargestellten Ausführungsbeispiel in Gestalt einer Feder oder eines Elastomerelementes, angeordnet. Die Kolbenstange 35 geht durch einen Kolben 31 hindurch, um aus dem Gehäuse 10 herausgeführt werden zu können.

Die durch den Arbeitskolben 30 getrennten Fluidkammern 41, 42 sind über Fluidleitungen 20, in denen zwei Stellventile 23, 23' und gegenläufig orientierte Rückschlagventile 24 angeordnet sind, hydraulisch miteinander gekoppelt. Ebenso sind die fluidgefüllten Nebenkammern 43, 44 über eine Hydraulikleitung mit gegenläufig orientierten Rückschlagventilen 24 miteinander verbunden. Die Verbindungsleitung der Nebenkammern 43, 44 ist zudem mit den Verbindungsleitungen zwischen den ersten Fluidkammern 41, 42 über eine Verbindungsleitung zwischen den beiden Rückschlagventilen 24 gekoppelt.

Die Ausführungsform stellt somit eine Kombination aus drei beweglichen Kolben 30, 31, 32 in drei Kammern und zwei Energiespeichern 50 in Gestalt von Federn dar. Die Energiespeicher 50 liegen außerhalb eines Fluidkontaktes an den Außenseiten der Federkolben 31, 32, während eine passive Verstellung des Arbeitskolbens über die Veränderung der Bewegungswiderstände oder Antriebe innen liegt. Die Durchgänge durch die Sperrwände 90 über die Stellventile 21, 22 oder die Rückschlagventile 24 können in den Sperrwänden 90 selbst angeordnet oder über außen an dem Gehäuse 10 entlanggeführte Kanäle mit entsprechenden Ventilen ausgeführt sein. Über die Stellventile 21, 22 und die Rückschlagventile 24 werden die Federkolben 31, 32 aus dem Kreislauf gesperrt oder eine Dämpfung bewirkt. Ebenfalls kann die passive Verstellung des Arbeitskolbens 30 durch die außerhalb des Gehäuses 10 angeordnete, parallel zu dem Arbeitskolben 30 verlaufende zwei-Wege-Verschaltung der Hydraulikleitung gesperrt werden.

Für den Fall, dass durch die außenliegenden Stellventile 23, 23' ein hydraulischer Ausgleich gesperrt wird und nur die Durchgänge zu den Federkolben 50 über die Ventile 21, 22 geöffnet sind, findet ein volumetrischer Ausgleich der Hydraulikflüssigkeit über die Federn statt. Das Volumen der fluidgefüllten Nebenkammern 43, 44 wird sich dabei je nach Stellung des Arbeitskolbens 30 ändern. Für den Fall, dass eine aktive Verstellung gesperrt ist und nur eine passive Verstellung über die außenliegenden Ventile 23, 23', 24 erfolgen soll, muss auf einen volumetrischen Ausgleich geachtet werden, hier müsste das entsprechende Stellventil 23, 23' jeweils geöffnet werden.

In der Figur 34 wird bei einer Ausfahrbewegung der Kolbenstange 35, beispielsweise bei einer Extension, der Arbeitskolben 30 in Pfeilrichtung bewegt. Dadurch wird der Druck in der oberen Fluidkammer 41 erhöht und Hochdruckfluid strömt gegen das erste Rückschlagventil 24 und durch das erste Stellventil 23, wie durch den Pfeil angedeutet, durch das zweite Stellventil 23' in die untere Fluidkammer 42. Ebenfalls gibt der untere Energiespeicher 50 Energie ab, indem er sich entspannt, wodurch Hydraulikfluid durch das untere Rückschlagventil 24 in die untere Fluidkammer 42 gefördert wird. Die Stellventile 21, 22 sind geschlossen, sodass sich eine über die Ventile 23, 23' gedämpfte Bewegung in Extensionsrichtung ergibt.

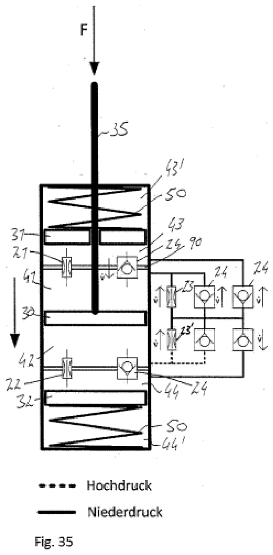

Bei einer Bewegungsumkehr, die in der Figur 35 gezeigt ist, sind die Stellventile 21, 22 in den Sperrwänden 90 weiterhin geschlossen, während die Stellventile 23, 23' in der außenliegenden Hydraulikschaltung geöffnet sind. Bei einer einfahrenden Bewegung der Kolbenstange 35, beispielsweise bei einer Flexion, strömt Hydraulikfluid von der unteren Fluidkammer 42 durch die Hochdruckleitung und das untere Stellventil 23', das obere Stellventil 23 sowie die beiden Rückschlagventile 24 sowohl in die obere Fluidkammer 41 des Arbeitskolbens 30 als auch in die obere Fluidkammer 43 des Federkolbens 31. Aufgrund des Druckes innerhalb der Verbindungsleitung zwischen dem Rückschlagventilen 24, die die Fluidkammern 43, 44 der Federkolben 31, 32 miteinander verbindet, wird gleichzeitig ein Fluidstrom in die Fluidkammer 44 des unteren Federkolbens 32 geleitet. Der obere Energiespeicher 50 entspannt sich, da er auf der Niederdruckseite angeordnet ist, sodass Hydraulikfluid aus der oberen Fluidkammer 43 durch das Rückschlagventil 24 in die obere Fluidkammer 41 des Arbeitskolbens 30 einströmen kann.

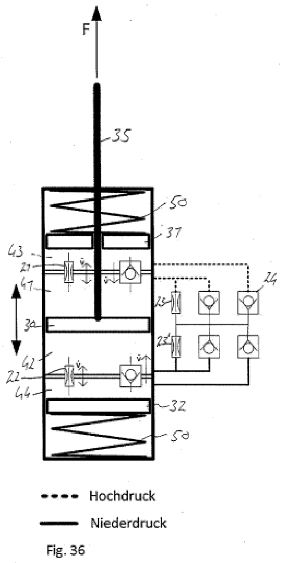

In der Figur 36 sind die Federventile 21, 22 geöffnet, während die externen Stellventile 23, 23' geschlossen sind. Durch die geöffneten Federventile 21, 22 ist eine hin- und hergehende Bewegung des Kolbens 30, wie durch den Doppelfeil angedeutet, möglich, da sich die Federn 50 in den Nebenkammern entspannen oder komprimiert werden. Der äußere Strömungsweg ist blockiert, sodass ein Volumenaustausch nur zwischen den jeweils benachbarten Fluidkammern 41, 43 und 42, 44 durch die Federventile 21, 22 und die Rückschlagventile 24 möglich ist.

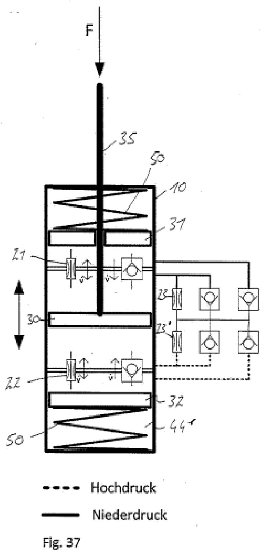

Figur 37 stellt die Situation dar, in der die Ventile analog zur Figur 36 geschaltet sind, jedoch eine Druckkraft auf die Kolbenstange 35 ausgeübt und der Arbeitskolben 30 nach unten gedrückt wird. Dementsprechend liegt die Hochdruckseite auf der Unterseite des Arbeitskolbens 30 und führt bei einer Kraftaufbringung nach unten, wie durch den Pfeil an der Kolbenstange 35 dargestellt, zu einem Einfedern der unteren Feder 50 bei einem Einfahren der Kolbenstange 35, beispielsweise bei einer Flexion und zu einem Entladen des oberen Energiespeichers 50. Entsprechend umgekehrt verhalten sich die Energiespeicher 50 bei einer umgekehrten Bewegung, also bei einem Ausfahren der Kolbenstange oder bei einer Extensionsbewegung, wie in der Figur 36 gezeigt.

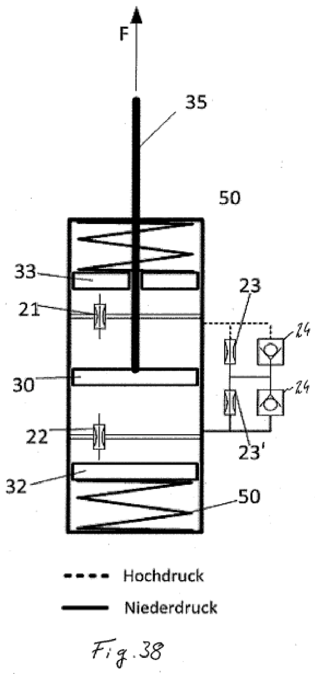

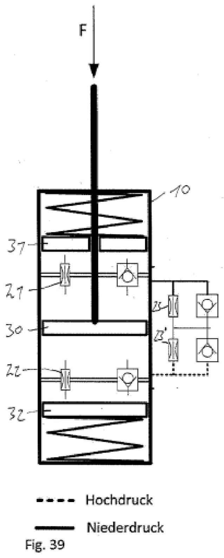

Figur 38 zeigt die Situation, bei alle Ventile 21, 22, 23, 23' geschlossen sind. Auch bei Aufbringung einer Zugkraft auf die Kolbenstange 35 ist eine Bewegung des Arbeitskolbens 30 blockiert, gleiches gilt für die umgekehrte Kraftaufbringung, die in der Figur 39 gezeigt ist.

Die Aktuator-Dämpfer-Einheit zum Einsatz in orthetischen oder prothetischen Vorrichtungen (1, 2, 3; 300), mit einem an der Vorrichtung (1, 2, 3, 300) festlegbaren Gehäuse (10), in dem ein Zylinder (12) ausgebildet ist, in dem ein erster Kolben (30) verlagerbar gelagert und mit einer Kolbenstange (35) gekoppelt ist, die mit einem ersten Ende (351) an dem ersten Kolben (30) angeordnet und mit einem zweiten Ende (352) mit der orthetischen oder prothetischen Vorrichtung (1, 2, 3, 300) koppelbar ist, wobei der erste Kolben (30) zwei Fluidkammern (41, 42) in dem Zylinder (12) voneinander trennt und eine Kolben-Zylinder-Einheit (100) bildet, ist dadurch gekennzeichnet, dass zumindest ein weiterer Kolben (32, 33) mit dem ersten Kolben (30) zur Ausbildung zumindest einer weiteren, volumenveränderbaren Fluidkammer (43, 44, 45, 46) gekoppelt ist.

Eine Ausführungsform der Aktuator-Dämpfer-Einheit ist dadurch gekennzeichnet, dass der erste Kolben (30) und der zumindest eine weitere Kolben (32, 33) hydraulisch miteinander gekoppelt sind.

Eine Ausführungsform der Aktuator-Dämpfer-Einheit ist dadurch gekennzeichnet, dass der weitere Kolben (32, 33) über einen komprimierbaren Energiespeicher (50) mit dem ersten Kolben (30) gekoppelt ist.

Eine Ausführungsform der Aktuator-Dämpfer-Einheit ist dadurch gekennzeichnet, dass der Energiespeicher (50) als Feder, Elastomerelement und/oder kompressibles Fluidvolumen ausgebildet ist.

Eine Ausführungsform der Aktuator-Dämpfer-Einheit ist dadurch gekennzeichnet, dass der Energiespeicher (50) über zumindest zwei Fluidleitungen verfügt, von denen zumindest eine mit einem Rückschlagventil ausgestattet ist.

Eine Ausführungsform der Aktuator-Dämpfer-Einheit ist dadurch gekennzeichnet, dass die zumindest eine weitere Fluidkammer (43, 44, 45, 46) hydraulisch mit der Kolben-Zylinder-Einheit (100) gekoppelt ist.

Eine Ausführungsform der Aktuator-Dämpfer-Einheit ist dadurch gekennzeichnet, dass der zumindest eine weitere Kolben (32, 33) in dem Zylinder (12) verlagerbar angeordnet ist.

Eine Ausführungsform der Aktuator-Dämpfer-Einheit ist dadurch gekennzeichnet, dass der zumindest eine weitere Kolben (32, 33) über eine Kolbenstange (35, 36) mit einem Teil einer orthetischen oder prothetischen Einrichtung (1, 2, 3; 300) verbunden ist.

Eine Ausführungsform der Aktuator-Dämpfer-Einheit ist dadurch gekennzeichnet, dass jede Fluidkammer (41, 42, 43, 44, 45, 46) zumindest eine Zugangsöffnung (411, 421, 431, 441) aufweist und über Fluidleitungen (20) mit zumindest einer anderen Fluidkammer (41, 42, 43, 44, 45, 46) verbunden ist.

Eine Ausführungsform der Aktuator-Dämpfer-Einheit ist dadurch gekennzeichnet, dass zumindest einer Fluidkammer (41, 42, 43, 44, 45, 46) zumindest ein Ventil (21, 21', 22, 22', 23, 23', 24, 25, 26, 27) zugeordnet ist, über das der Fluidstrom in und aus der Fluidkammer (41, 42, 43, 44, 45, 46) einstellbar ist und/oder dass eine die Viskosität des Fluides beeinflussende Einrichtung der Aktuator-Dämpfer-Einheit zugeordnet ist.

Eine Ausführungsform der Aktuator-Dämpfer-Einheit ist dadurch gekennzeichnet, dass das Ventil (21, 21', 22, 22', 23, 23', 24, 25, 26, 27) als Schaltventil, Stellventil oder Rückschlagventil ausgebildet ist.

Eine Ausführungsform der Aktuator-Dämpfer-Einheit ist dadurch gekennzeichnet, dass zumindest ein Ausgleichsvolumen (60) mit zumindest einer der Fluidkammern (41, 42, 43, 44, 45, 46) gekoppelt ist.

Eine Ausführungsform der Aktuator-Dämpfer-Einheit ist dadurch gekennzeichnet, dass das Ausgleichsvolumen (60) über zumindest ein Ventil (21, 21', 22, 22', 23, 23', 24, 25, 26, 27) mit der Fluidkammer (41, 42, 43, 44, 45, 46) gekoppelt ist.

Eine Ausführungsform der Aktuator-Dämpfer-Einheit ist dadurch gekennzeichnet, dass das Ausgleichsvolumen (60) als Druckspeicher ausgebildet ist.

Eine Ausführungsform der Aktuator-Dämpfer-Einheit ist dadurch gekennzeichnet, dass das Ausgleichsvolumen (60) in einer Fluidkammer (43, 44, 45, 46) mit einem Energiespeicher (50) integriert ist.

Eine Ausführungsform der Aktuator-Dämpfer-Einheit ist dadurch gekennzeichnet, dass eine Pumpe (70) zur Erhöhung des Fluiddruckes der Aktuator-Dämpfer-Einheit zugeordnet ist.

Eine Ausführungsform der Aktuator-Dämpfer-Einheit ist dadurch gekennzeichnet, dass die Pumpe (70) einer zweiten Kolben-Zylinder-Einheit (102) zugeordnet ist, die gleichgerichtet zu der ersten Kolben-Zylinder-Einheit (100) geschaltet ist.

Eine Ausführungsform der Aktuator-Dämpfer-Einheit ist dadurch gekennzeichnet, dass die Pumpe (70) mit einem Energiespeicher (50) gekoppelt ist.

Eine Ausführungsform der Aktuator-Dämpfer-Einheit ist dadurch gekennzeichnet, dass das Fluid als Hydraulikfluid ausgebildet ist.

Eine Ausführungsform der Aktuator-Dämpfer-Einheit ist dadurch gekennzeichnet, dass eine Steuereinrichtung (80) den Ventilen (21, 21', 22, 22', 23, 23', 24, 25, 26, 27) zu deren Verstellung oder Schaltung zugeordnet ist.

Eine Ausführungsform der Aktuator-Dämpfer-Einheit ist dadurch gekennzeichnet, dass die Steuereinrichtung (80) mit zumindest einem Sensor (85) der orthetischen oder prothetischen Vorrichtung (1, 2, 3; 300) gekoppelt ist, der Zustandsdaten der Aktuator-Dämpfer-Einheit und/oder der orthetischen oder prothetischen Vorrichtung (1, 2, 3; 300) der Steuereinrichtung (80) übermittelt.

Eine Ausführungsform der Aktuator-Dämpfer-Einheit ist dadurch gekennzeichnet, dass der zumindest eine Sensor (85) zur Ermittlung einer Kolbenposition, einer aufgebrachten Kraft und/oder eines Druckes ausgebildet ist.

Eine Ausführungsform der Aktuator-Dämpfer-Einheit ist dadurch gekennzeichnet, dass zwei weitere Kolben (32, 33) in dem Zylinder (12) angeordnet oder mit dem ersten Kolben (30) gekoppelt sind, die zwei weitere, volumenveränderbare Fluidkammern (43, 44, 45, 46) ausbilden.

Eine Ausführungsform der Aktuator-Dämpfer-Einheit ist dadurch gekennzeichnet, dass die beiden weiteren Kolben (32, 33) auf einander gegenüberliegenden Seiten des ersten Kolbens (30) angeordnet sind.

Eine Ausführungsform der Aktuator-Dämpfer-Einheit ist dadurch gekennzeichnet, dass die beiden weiteren Kolben (32, 33) in von dem Zylinder (12) strömungstechnisch entkoppelten Zylindern (14, 15) angeordnet sind.

Eine Ausführungsform der Aktuator-Dämpfer-Einheit ist dadurch gekennzeichnet, dass ein weiterer Kolben (33) in einem relativ zu dem Gehäuse (10) des ersten Kolbens (30) verlagerbaren zweiten Gehäuse (10') gelagert ist.

## Patentansprüche

1. Aktuator-Dämpfer-Einheit zum Einsatz in orthetischen oder prothetischen Vorrichtungen (1, 2, 3; 300), mit einem an der Vorrichtung (1, 2, 3, 300) festlegbaren Gehäuse (10), in dem ein Zylinder (12) ausgebildet ist, in dem ein erster Kolben (30) verlagerbar gelagert und mit einer Kolbenstange (35) gekoppelt ist, die mit einem ersten Ende (351) an dem ersten Kolben (30) angeordnet und mit einem zweiten Ende (352) mit der orthetischen oder prothetischen Vorrichtung (1, 2, 3, 300) koppelbar ist, wobei der erste Kolben (30) zwei Fluidkammern (41, 42) in dem Zylinder (12) voneinander trennt und eine Kolben-Zylinder-Einheit (100) bildet, **dadurch gekennzeichnet, dass** zumindest ein weiterer Kolben (32, 33) in dem Zylinder (12) verlagerbar angeordnet und mit dem ersten Kolben (30) zur Ausbildung zumindest einer weiteren, volumenveränderbaren Fluidkammer (43, 44, 45, 46) über einen komprimierbaren Energiespeicher (50) zwischen den Kolben (30, 32, 33) gekoppelt ist.

2. Aktuator-Dämpfer-Einheit nach Anspruch 1, **dadurch gekennzeichnet, dass** der Energiespeicher (50) als Feder, Elastomerelement und/oder kompressibles Fluidvolumen ausgebildet ist.

3. Aktuator-Dämpfer-Einheit nach Anspruch 1 oder 2, **dadurch gekennzeichnet, dass** der Energiespeicher (50) über zumindest zwei Fluidleitungen verfügt, von denen zumindest eine mit einem Rückschlagventil ausgestattet ist.

4. Aktuator-Dämpfer-Einheit nach einem der voranstehenden Ansprüche, **dadurch gekennzeichnet, dass** die zumindest eine weitere Fluidkammer (43, 44, 45, 46) hydraulisch mit der Kolben-Zylinder-Einheit (100) gekoppelt ist.

5. Aktuator-Dämpfer-Einheit nach einem der voranstehenden Ansprüche, **dadurch gekennzeichnet, dass** der zumindest eine weitere Kolben (32, 33) über eine Kolbenstange (35, 36) mit einem Teil einer orthetischen oder prothetischen Einrichtung (1, 2, 3; 300) verbunden ist.

6. Aktuator-Dämpfer-Einheit nach einem der voranstehenden Ansprüche, **dadurch gekennzeichnet, dass** zumindest einer Fluidkammer (41, 42, 43, 44, 45, 46) zumindest ein Ventil (21, 21', 22, 22', 23, 23', 24, 25, 26, 27) zugeordnet ist, über das der Fluidstrom in und aus der Fluidkammer (41, 42, 43, 44, 45, 46) einstellbar ist.

7. Aktuator-Dämpfer-Einheit nach Anspruch 6, **dadurch gekennzeichnet, dass** das Ventil (21, 21', 22, 22', 23, 23', 24, 25, 26, 27) als Schaltventil, Stellventil oder Rückschlagventil ausgebildet ist.

8. Aktuator-Dämpfer-Einheit nach einem der voranstehenden Ansprüche, **dadurch gekennzeichnet, dass** zumindest ein Ausgleichsvolumen (60) mit zumindest einer der Fluidkammern (41, 42, 43, 44, 45, 46) gekoppelt ist.

9. Aktuator-Dämpfer-Einheit nach Anspruch 8, **dadurch gekennzeichnet, dass** das Ausgleichsvolumen (60) über zumindest ein Ventil (21, 21', 22, 22', 23, 23', 24, 25, 26, 27) mit der Fluidkammer (41, 42, 43, 44, 45, 46) gekoppelt ist.

10. Aktuator-Dämpfer-Einheit nach einem der Ansprüche 8 oder 9, **dadurch gekennzeichnet, dass** das Ausgleichsvolumen (60) als Druckspeicher ausgebildet ist.

11. Aktuator-Dämpfer-Einheit nach einem der Ansprüche 8 bis 10, **dadurch gekennzeichnet, dass** das Ausgleichsvolumen (60) in einer Fluidkammer (43, 44, 45, 46) mit einem Energiespeicher (50) integriert ist.

12. Aktuator nach einem der voranstehenden Ansprüche, **dadurch gekennzeichnet, dass** eine Pumpe (70) zur Erhöhung des Fluiddruckes der Aktuator-Dämpfer-Einheit zugeordnet ist.

13. Aktuator-Dämpfer-Einheit nach einem der Ansprüche 6, 7 oder 9, **dadurch gekennzeichnet, dass** eine Steuereinrichtung (80) den Ventilen (21, 21', 22, 22', 23, 23', 24, 25, 26, 27) zu deren Verstellung oder Schaltung zugeordnet ist.

14. Aktuator-Dämpfer-Einheit nach Anspruch 13, **dadurch gekennzeichnet, dass** die Steuereinrichtung (80) mit zumindest einem Sensor (85) der orthetischen oder prothetischen Vorrichtung (1, 2, 3; 300) gekoppelt ist, der Zustandsdaten der Aktuator-Dämpfer-Einheit und/oder der orthetischen oder prothetischen Vorrichtung (1, 2, 3; 300) der Steuereinrichtung (80) übermittelt.

15. Aktuator-Dämpfer-Einheit nach einem der voranstehenden Ansprüche, **dadurch gekennzeichnet, dass** zwei weitere Kolben (32, 33) in dem Zylinder (12) angeordnet oder mit dem ersten Kolben (30) gekoppelt sind, die zwei weitere, volumenveränderbare Fluidkammern (43, 44, 45, 46) ausbilden.
